# EUROPEAN PATENT APPLICATION

(11) **EP 4 468 401 A1**
(43) Date of publication of application: **27.11.2024**
(21) Application number: 23740227.6
(22) Date of filing: 06.01.2023
(51) Int. Cl.: H01M 4/139, H01G 11/06, H01G 11/30, H01G 11/36, H01G 11/38, H01M 4/13, H01M 4/485, H01M 4/62

(54) **COMPOSITION, ELECTRODE, AND SECONDARY BATTERY**

(30) Priority: 17.01.2022 JP 2022005318
(71) Applicant: DAIKIN INDUSTRIES, LTD., Osaka-shi, Osaka 530-0001 (JP); MCD Technologies S.a.r.l, 3364 Leudelange (LU)
(72) Inventor: HOSODA, Chihiro, Osaka-Shi, Osaka 530-0001 (JP); YAMAZAKI, Shigeaki, Osaka-Shi, Osaka 530-0001 (JP); TERADA, Junpei, Osaka-Shi, Osaka 530-0001 (JP); SHIMOOKA, Toshiharu, Osaka-Shi, Osaka 530-0001 (JP); ICHINOSE, Yuma, Osaka-Shi, Osaka 530-0001 (JP); NISHIYAMA, Yumi, Osaka-Shi, Osaka 530-0001 (JP); PREDTECHENSKIY, Mikhail Rudolfovich, Novosibirsk 630090 (RU); BOBRENOK, Oleg Filippovich, Novosibirsk 630090 (RU)
(74) Representative: Hoffmann Eitle
(86) International application number: PCT/JP2023/000202
(87) International publication number: WO 2023/136218

(57) **Abstract**

Provided is a composition, comprising single-walled carbon nanotube, a binder, and a specific solvent, wherein the binder contains a fluorine-containing polymer containing a vinylidene fluoride unit.

## Description

### TECHNICAL FIELD

The present disclosure relates to a composition, an electrode, and a secondary battery.

### BACKGROUND ART

An electrode that an electrochemical device such as a lithium ion battery includes can be formed, for example, by dissolving an electrode material and a binder in a solvent and applying the obtained composition to a current collector.

As such a composition, Patent Document 1 describes a composition for an electrochemical device, containing a single-walled carbon nanotube, a binder, and a solvent, in which the binder contains a fluorine-containing copolymer containing vinylidene fluoride unit and a fluorinated monomer unit, excluding the vinylidene fluoride unit, and a content of the vinylidene fluoride unit in the fluorine-containing copolymer is 50.0 mol% or more, based on all monomer units.

### RELATED ART

### PATENT DOCUMENTS

Patent Document 1: International Publication No. WO 2021/002369

### SUMMARY OF THE INVENTION

### PROBLEM TO BE SOLVED BY THE INVENTION

An object of the present disclosure is to provide a composition that allows a battery having excellent output characteristics, cycle characteristics, and 60°C storage characteristics and having a reduced gas generation rate after high-temperature preservation to be obtained.

### MEANS FOR SOLVING THE PROBLEM

According to the present disclosure, provided is a composition, containing a single-walled carbon nanotube, a binder, and a solvent represented by a general formula (1), wherein the binder contains a fluorine-containing polymer containing vinylidene fluoride unit. 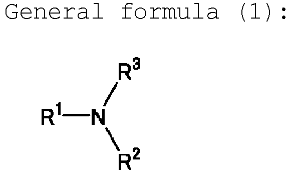 wherein R¹, R², and R³ are each independently H or a monovalent substituent, provided that the total number of carbon atoms of R¹, R², and R³ is 6 or more, and at least one of R¹, R², and R³ is an organic group having carbonyl group; and any two of R¹, R², and R³ optionally bond to each other to form a ring.

The solvent is preferably a solvent represented by a general formula (1a): 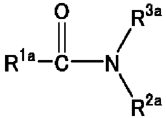 wherein R^{1a} is a monovalent substituent, R^{2a} and R^{3a} are each independently H or a monovalent substituent, provided that the total number of carbon atoms of R^{1a}, R^{2a}, and R^{3a} is 5 or more; and any two of R^{1a}, R^{2a}, and R^{3a} optionally bond to each other to form a ring.

The solvent is preferably at least one selected from the group consisting of a solvent represented by a general formula (1b-1) and a solvent represented by a general formula (1b-2). 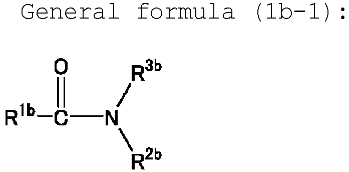 wherein R^{1b} is an alkyl group, an alkoxyalkyl group, an acylalkyl group, an alkenyl group, an amino group, or an aminoalkyl group, R^{2b} and R^{3b} are each independently an alkyl group or an alkoxyalkyl group, the total number of carbon atoms of R^{1b}, R^{2b}, and R^{3b} is 5 or more, R^{2b} and R^{3b} optionally bond to each other to form a ring with a nitrogen atom to which R^{2b} and R^{3b} bond, and an oxygen atom is optionally contained as a ring-constituting atom. 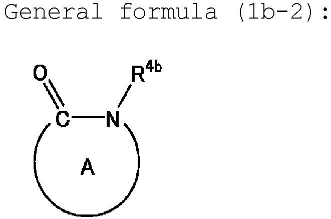 wherein a ring A is a 5-membered or 6-membered amide ring, R^{4b} is an alkyl group, a cycloalkyl group, or an alkenyl group, and the total number of carbon atoms of the ring A and R^{4b} is 5 or more.

The solvent is preferably at least one selected from the group consisting of 3-methoxy-N,N-dimethylpropanamide, N-ethyl-2-pyrrolidone, and N-butyl-2-pyrrolidone.

An average outer diameter of the single-walled carbon nanotube is preferably 2.5 nm or less.

An average G/D ratio of the single-walled carbon nanotube is preferably 2 or more.

The binder preferably contains a polyvinylidene fluoride as the fluorine-containing polymer.

The binder preferably contains a fluorine-containing copolymer containing vinylidene fluoride unit and a unit of an other monomer other than vinylidene fluoride as the fluorine-containing polymer.

The other monomer is preferably a fluorinated monomer, excluding vinylidene fluoride.

The other monomer is preferably at least one selected from the group consisting of tetrafluoroethylene, chlorotrifluoroethylene, (meth)acrylic acid, 2,3,3,3-tetrafluoropropene, hexafluoropropylene, and a fluoroalkyl vinyl ether.

The binder preferably contains a polyvinylidene fluoride and a fluorine-containing copolymer containing vinylidene fluoride unit and a unit of an other monomer other than vinylidene fluoride, as the fluorine-containing polymer.

A mass ratio of the polyvinylidene fluoride to the fluorine-containing copolymer, polyvinylidene fluoride/fluorine-containing polymer, is preferably 99/1 to 1/99.

The composition of the present disclosure can be used to form an electrode of an electrochemical device.

The composition of the present disclosure preferably further contains a powder electrode material, excluding the single-walled carbon nanotube.

The powder electrode material preferably contains a lithium composite oxide as a positive electrode active material.

The powder electrode material preferably contains a negative electrode active material that exhibits a potential of 2.5 V or less vs. Li when alloyed with Li or bonded with Li.

In addition, according to the present disclosure, provided is an electrode including a current collector and an electrode material layer provided on one side or each of both sides of the current collector and formed from the composition.

In addition, according to the present disclosure, a secondary battery including the above electrode is provided.

### EFFECTS OF INVENTION

According to the present disclosure, it is possible to provide a composition that allows a battery having excellent output characteristics, cycle characteristics, and 60°C storage characteristics and having a reduced gas generation rate after high-temperature preservation to be obtained.

### DESCRIPTION OF EMBODIMENTS

Hereinafter, specific embodiments of the present disclosure will be described in detail, but the present disclosure is not limited to the following embodiments.

The composition of the present disclosure contains a single-walled carbon nanotube, a binder, and a solvent.

Patent Document 1 describes that, by forming an electrode using the composition for an electrochemical device containing a single-walled carbon nanotube, a specific binder, and a solvent, it is possible to obtain a secondary battery having excellent output characteristics, cycle characteristics, and 60°C storage characteristics.

As a result of intensive studies of means for further improving the performance of a secondary battery, it was found that, by forming an electrode using the composition containing an extremely limited amount of a solvent, it is possible to obtain a secondary battery not only having excellent output characteristics, but also having further improved cycle characteristics and 60°C storage characteristics and a reduced gas generation rate after high-temperature preservation. Hereinafter, each component that the composition of the present disclosure contains will be described in detail.

### (Solvent)

The composition of the present disclosure contains a solvent represented by a general formula (1) together with a single-walled carbon nanotube and a binder. The solvent that is used in the present disclosure is suitably a solvent that is liquid at 20°C. 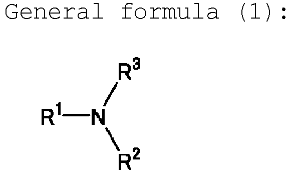 wherein R¹, R², and R³ are each independently H or a monovalent substituent, provided that the total number of carbon atoms of R¹, R², and R³ is 6 or more, and at least one of R¹, R², and R³ is an organic group having a carbonyl group; and any two of R¹, R², and R³ optionally bond to each other to form a ring.

The total number of carbon atoms of R¹, R², and R³ is 6 or more. That is, the kinds of the groups of R¹, R², and R³ are each selected so that the total number of carbon atoms becomes 6 or more. The upper limit of the total number of carbon atoms of R¹, R², and R³ is not limited and may be 16 or less, 14 or less, or 12 or less.

R¹, R², and R³ are each independently H or a monovalent substituent. The monovalent substituent is preferably an alkyl group, an alkoxyalkyl group, an acylalkyl group, an alkenyl group, an amino group, an aminoalkyl group, or a cycloalkyl group.

At least one of R¹, R², and R³ is an organic group having a carbonyl group. The organic group having a carbonyl group is preferably an acyl group. The acyl group is preferably a group represented by a general formula: -CO-R⁴ wherein R⁴ is an alkyl group having 1 to 6 carbon atoms. When the number of carbon atoms of the alkyl group is 3 or more, the alkyl group may be linear or branched in some embodiments. When the number of carbon atoms of the alkyl group as R⁴ is 2 or more, the alkyl group optionally includes a heteroatom such as an oxygen atom or a nitrogen atom or a carbonyl group between carbon atoms thereof.

Any two of R¹, R², and R³ optionally bond to each other to form a ring. In addition, R¹, R², and R³ optionally include a heteroatom such as an oxygen atom as a ring-constituting atom. The ring is preferably a saturated ring. The number of members of the ring is not limited, but is preferably 5 or 6. The ring is preferably a pyrrolidine ring, an oxazoline ring, a piperidine ring, or a morpholine ring.

The solvent is preferably a solvent represented by a general formula (1a). 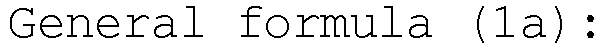 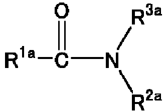 wherein R^{1a} is a monovalent substituent, R^{2a} and R^{3a} are each independently H or a monovalent substituent, provided that the total number of carbon atoms of R^{1a}, R^{2a}, and R^{3a} is 5 or more; and any two of R^{1a}, R^{2a}, and R^{3a} optionally bond to each other to form a ring.

In the general formula (1a), the total number of carbon atoms of R^{1a}, R^{2a}, and R^{3a} is 5 or more. That is, the kinds of the groups of R^{1a}, R^{2a}, and R^{3a} are each selected so that the total number of carbon atoms becomes 5 or more. The upper limit of the total number of carbon atoms of R^{1a}, R^{2a}, and R^{3a} is not limited and may be 15 or less, 13 or less, or 11 or less.

In the general formula (1a), R^{1a} is a monovalent substituent. The monovalent substituent is preferably an alkyl group, an alkoxyalkyl group, an acylalkyl group, an alkenyl group, an amino group, an aminoalkyl group, or a cycloalkyl group, and more preferably an alkyl group, an alkoxyalkyl group, an acylalkyl group, an alkenyl group, an amino group, or an aminoalkyl group.

In the general formula (1a), R^{2a} and R^{3a} are each independently H or a monovalent substituent. R^{2a} and R^{3a} are preferably each independently a monovalent substituent. The monovalent substituent is preferably an alkyl group, an alkoxyalkyl group, an acylalkyl group, an alkenyl group, an amino group, an aminoalkyl group, or a cycloalkyl group, and more preferably an alkyl group, an alkoxyalkyl group, a cycloalkyl group, or an alkenyl group.

Any two of R^{1a}, R^{2a}, and R^{3a} optionally bond to each other to form a ring. In particular, R^{2a} and R^{3a} preferably bond to each other to form a ring together with a nitrogen atom to which R^{2a} and R^{3a} bond. In addition, R^{1a}, R^{2a}, and R^{3a} optionally include a heteroatom such as an oxygen atom as a ring-constituting atom. The ring is preferably a saturated ring. The number of members of the ring is not limited, but is preferably 5 or 6. The ring is preferably a pyrrolidine ring, an oxazoline ring, a piperidine ring, or a morpholine ring.

The solvent is preferably at least one selected from the group consisting of a solvent represented by a general formula (1b-1) and a solvent represented by a general formula (1b-2). 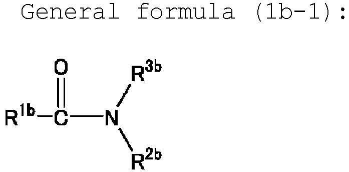 wherein R^{1b} is an alkyl group, an alkoxyalkyl group, an acylalkyl group, an alkenyl group, an amino group, or an aminoalkyl group, R^{2b} and R^{3b} are each independently an alkyl group or an alkoxyalkyl group, the total number of carbon atoms of R^{1b}, R^{2b}, and R^{3b} is 5 or more, R^{2b} and R^{3b} optionally bond to each other to form a ring with a nitrogen atom to which R^{2b} and R^{3b} bond, and an oxygen atom is optionally contained as a ring-constituting atom. 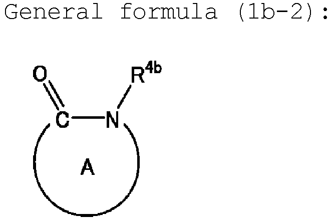 wherein a ring A is a 5-membered or 6-membered amide ring, R^{4b} is an alkyl group, a cycloalkyl group, or an alkenyl group, and the total number of carbon atoms of the ring A and R^{4b} is 5 or more.

In the general formula (1b-1), the total number of carbon atoms of R^{1b}, R^{2b}, and R^{3b} is 5 or more. That is, the kinds of the groups of R^{1b}, R^{2b}, and R^{3b} are each selected so that the total number of carbon atoms becomes 5 or more. The upper limit of the total number of carbon atoms of R^{1b}, R^{2b}, and R^{3b} is not limited and may be 15 or less, 13 or less, or 11 or less.

In the general formula (1b-1), R^{1b} is an alkyl group, an alkoxyalkyl group, an acylalkyl group, an alkenyl group, an amino group, or an aminoalkyl group.

The alkyl group as R^{1b} is preferably an alkyl group having 1 to 10 carbon atoms. When the number of carbon atoms of the alkyl group is 3 or more, the alkyl group may be linear or branched in some embodiments.

The alkoxyalkyl group as R^{1b} is preferably a group represented by a general formula: -R^{1b1}-O-R^{1b2} wherein R^{1b1} is an alkylene group having 1 to 5 carbon atoms, and R^{1b2} is an alkyl group having 1 to 5 carbon atoms. When the numbers of carbon atoms of the alkyl group and the alkylene group are 3 or more, the alkyl group and the alkylene group may be linear or branched in some embodiments.

The acylalkyl group as R^{1b} is preferably a group represented by a general formula: -R^{1b3}-CO-R^{1b4} wherein R^{1b3} is an alkylene group having 1 to 5 carbon atoms, and R^{1b4} is an alkyl group having 1 to 5 carbon atoms. When the numbers of carbon atoms of the alkyl group and the alkylene group are 3 or more, the alkyl group and the alkylene group may be linear or branched in some embodiments.

The alkenyl group as R^{1b} is preferably a group represented by a general formula: -R^{1b5}-CR^{1b6}=CR^{1b7} wherein R^{1b5} is a single bond or an alkylene group having 1 to 5 carbon atoms, and R^{1b6} and R^{1b7} are each independently H or an alkyl group having 1 to 5 carbon atoms. When the numbers of carbon atoms of the alkyl group and the alkylene group are 3 or more, the alkyl group and the alkylene group may be linear or branched in some embodiments. The alkenyl group is preferably a vinyl group.

The amino group as R^{1b} and an amino group that the aminoalkyl group has is a monovalent functional group obtained by removing hydrogen from ammonia or a primary or secondary amine. When R^{1b} is the amino group, the amino group can form an amide bond together with a carbonyl group to which R^{1b} bonds.

The amino group as R^{1b} is preferably a group represented by a general formula: -N-(R^{1b8})₂ wherein R^{1b8} is H or an alkyl group having 1 to 5 carbon atoms. When the number of carbon atoms of the alkyl group is 3 or more, the alkyl group may be linear or branched in some embodiments. The amino group is preferably -N-(CH₃)₂ or -N-(C₂H₅)₂.

The aminoalkyl group as R^{1b} is preferably a group represented by a general formula: -R^{1b9}-N-(R^{1b8})₂ wherein R^{1b9} is an alkylene group having 1 to 5 carbon atoms and R^{1b8} is H or an alkyl group having 1 to 5 carbon atoms. When the numbers of carbon atoms of the alkyl group and the alkylene group are 3 or more, the alkyl group and the alkylene group may be linear or branched in some embodiments.

In the general formula (1b-1), R^{2b} and R^{3b} are each independently an alkyl group or an alkoxyalkyl group.

The alkyl group as R^{2b} and R^{3b} is preferably an alkyl group having 1 to 10 carbon atoms. When the number of carbon atoms of the alkyl group is 3 or more, the alkyl group may be linear or branched in some embodiments.

The alkoxyalkyl group as R^{2b} and R^{3b} is preferably a group represented by a general formula: -R^{2b1}-N-R^{2b2} wherein R^{2b1} is an alkylene group having 1 to 5 carbon atoms and R^{2b2} is an alkyl group having 1 to 5 carbon atoms. When the numbers of carbon atoms of the alkyl group and the alkylene group are 3 or more, the alkyl group and the alkylene group may be linear or branched in some embodiments.

R^{2b} and R^{3b} optionally bond to each other to form a ring together with a nitrogen atom to which R^{2b} and R^{3b} bond and optionally include an oxygen atom as a ring-constituting atom. The ring is preferably a saturated ring. The number of members of the ring is not limited, but is preferably 5 or 6. The ring is preferably a pyrrolidine ring, an oxazoline ring, a piperidine ring, or a morpholine ring.

In the general formula (1b-2), the total number of carbon atoms of the ring A and R^{4b} is 5 or more. That is, the kinds of the ring and the group of the ring A and R^{4b} are selected so that the total number of carbon atoms becomes 5 or more. The upper limit of the total number of carbon atoms of the ring A and R^{4b} is not limited and may be 15 or less, 13 or less, or 11 or less.

The ring A is a 5-membered or 6-membered amide ring. Therefore, the ring A is composed of an alkylene group having 3 or 4 carbon atoms together with a carbon atom and a nitrogen atom. A hydrogen atom of the alkylene group constituting the ring A that bonds to the carbon atom may be substituted with a substituent in some embodiments or may not be substituted with a substituent in other embodiments, but is preferably not substituted with a substituent. Examples of the substituent include alkyl groups such as a methyl group.

R^{4b} is an alkyl group, a cycloalkyl group, or an alkenyl group.

The alkyl group as R^{4b} is preferably an alkyl group having 1 to 10 carbon atoms. When the number of carbon atoms of the alkyl group is 3 or more, the alkyl group may be linear or branched in some embodiments.

The cycloalkyl group as R^{4b} is preferably a cycloalkyl group having 3 to 10 carbon atoms. The cycloalkyl group is preferably a cyclobutyl group, a cyclopentyl group, a cyclohexyl group, a cyclopropyl group, or a cyclooctyl group.

The alkenyl group as R^{4b} is preferably a group represented by a general formula: -R^{4b1}-CR^{4b2}=CR^{4b3} wherein R^{4b1} is a single bond or an alkylene group having 1 to 5 carbon atoms and R^{4b2} and R^{4b4} are each independently H or an alkyl group having 1 to 5 carbon atoms. When the numbers of carbon atoms of the alkyl group and the alkylene group are 3 or more, the alkyl group and the alkylene group may be linear or branched in some embodiments. The alkenyl group is preferably a vinyl group.

The solvent is preferably at least one selected from the group consisting of 3-methoxy-N,N-dimethylpropanamide, N-ethyl-2-pyrrolidone (NEP), N-butyl-2-pyrrolidone (NBP), acryloylmorpholine, N-cyclohexyl-2-pyrrolidone, N-vinyl-2-pyrrolidone, 3-butoxy-N,N-dimethylpropanamide, N,N,N',N'-tetraethylurea, N,N-dimethylacetoacetamide, N-octyl-2-pyrrolidone, and N,N-diethylacetamide.

Among these, the solvent is more preferably at least one selected from the group consisting of 3-methoxy-N,N-dimethylpropanamide, N-ethyl-2-pyrrolidone, and N-butyl-2-pyrrolidone.

The amount of the solvent in the composition is determined in consideration of coating of the current collector, formation of a thin film after drying, and the like. Usually, the proportion by mass of the binder to the solvent is 0.5:99.5 to 20:80.

### (Single-walled carbon nanotubes)

The single-walled carbon nanotubes (SWCNTs) are a special kind of carbon material, which is known as a one-dimensional material. The single-walled carbon nanotube is made of a graphene sheet and has been wound to form a hollow tube having a wall as thick as one atom. The single-walled carbon nanotube has such a chemical structure and size and thereby exhibits excellent mechanical, electrical, thermal, and chemical characteristics. The composition of the present disclosure contains the single-walled carbon nanotubes, and use of the composition of the present disclosure thus allows a battery having excellent output characteristics, cycle characteristics, and 60°C storage characteristics and having a reduced gas generation rate to be obtained.

The average outer diameter of the single-walled carbon nanotubes is preferably 2.5 nm or less, more preferably 2.0 nm or less, further preferably 1.8 nm or less, and preferably 1.0 nm or more, more preferably 1.1 nm or more, further preferably 1.2 nm or more. The average outer diameter of the single-walled carbon nanotubes can be determined from the light absorption spectrum or Raman spectrum obtained by ultraviolet-visible-near infrared spectroscopy (UV-Vis-NIR) or a transmission electron microscope (TEM) image.

The average G/D ratio measured by the Raman spectroscopy (wavelength: 532 nm) of the single-walled carbon nanotubes is preferably 2 or more, more preferably 5 or more, further preferably 10 or more, particularly preferably 30 or more, most preferably 40 or more and preferably 250 or less, more preferably 220 or less, further preferably 180 or less. The G/D ratio refers to the intensity ratio (G/D) of the G band to the D band of the Raman spectrum of the single-walled carbon nanotubes. As the average G/D ratio of the single-walled carbon nanotubes becomes higher, it means that the crystallinity of the single-walled carbon nanotubes becomes higher and the number of carbon nanotubes having impurity carbon or defects becomes smaller.

The average length of the single-walled carbon nanotubes is preferably 0.1 to 50 µm, more preferably 0.5 to 20 µm, and further preferably 1 to 10 µm. The average length of the single-walled carbon nanotubes can be determined by obtaining an AFM image of the single-walled carbon nanotubes using an atomic force microscope (AFM) or obtaining a TEM image of the single-walled carbon nanotubes using a transmission electron microscope (TEM), measuring the length of each single-walled carbon nanotube, and dividing the total value of the lengths by the number of the measured single-walled carbon nanotubes.

The content of the single-walled carbon nanotube in the composition of the present disclosure is preferably 0.01 to 3% by mass, more preferably 0.01 to 2% by mass, further preferably 0.01 to 1% by mass, particularly preferably 0.1 to 0.8% by mass, and most preferably 0.2 to 0.5% by mass, based on the mass of the composition. When the content of the single-walled carbon nanotube is within the above range, the composition has an appropriate viscosity, and it is possible to prepare an electrode mixture in which each component has sufficiently dispersed without imparting an excess shear force. Therefore, it is possible to sufficiently form a three-dimensional network structure of the single-walled carbon nanotubes in the obtained electrode material layer.

### (Binder)

The binder that is contained in the composition of the present disclosure contains a fluorine-containing polymer containing vinylidene fluoride (VdF) unit.

The fluorine-containing polymer is preferably at least one selected from the group consisting of a polyvinylidene fluoride (PVdF) and a fluorine-containing copolymer containing VdF unit and a unit of an other monomer other than VdF.

The fluorine-containing copolymer contains VdF unit and a unit of an other monomer other than VdF. The other monomer may be a fluorinated monomer or a non-fluorinated monomer in some embodiments.

Examples of the fluorinated monomer, excluding VdF, include tetrafluoroethylene (TFE), vinyl fluoride, trifluoroethylene, chlorotrifluoroethylene (CTFE), fluoroalkyl vinyl ether, hexafluoropropylene (HFP), a (perfluoroalkyl)ethylene, 2,3,3,3-tetrafluoropropene, and trans-1,3,3,3-tetrafluoropropene.

The fluoroalkyl vinyl ether is preferably a fluoroalkyl vinyl ether having a fluoroalkyl group having 1 to 5 carbon atoms, and more preferably at least one selected from the group consisting of perfluoro(methyl vinyl ether) (PMVE), perfluoro(ethyl vinyl ether), and perfluoro(propyl vinyl ether).

The fluorinated monomer is preferably at least one selected from the group consisting of TFE, CTFE, 2,3,3,3-tetrafluoropropene, HFP, and fluoroalkyl vinyl ether, more preferably at least one selected from the group consisting of TFE and HFP, and particularly preferably TFE.

The fluorinated monomer, excluding VdF, may or may not have a polar group.

Examples of the non-fluorinated monomer include a non-fluorinated monomer having no polar group such as ethylene or propylene, and a non-fluorinated monomer having a polar group (hereinafter, sometimes referred to as a polar group-containing monomer).

When a non-fluorinated monomer having a polar group is used, the polar group is introduced into the fluorine-containing copolymer, and this allows far superior adhesion between a coating layer and a metal foil to be obtained. The polar group is preferably at least one selected from the group consisting of a carbonyl group-containing group, an epoxy group, a hydroxy group, a sulfonic acid group, a sulfuric acid group, a phosphoric acid group, an amino group, an amide group, and an alkoxy group, more preferably at least one selected from the group consisting of a carbonyl group-containing group, an epoxy group, and a hydroxy group, and further preferably a carbonyl group-containing group. The hydroxy group does not include a hydroxy group constituting a part of the carbonyl group-containing group. In addition, the amino group is a monovalent functional group obtained by removing hydrogen from ammonia or a primary or secondary amine.

The carbonyl group-containing group is a functional group having a carbonyl group (-C(=O)-). The carbonyl group-containing group is preferably a group represented by the general formula: - COOR wherein R represents a hydrogen atom, an alkyl group, or a hydroxyalkyl group, or a carboxylic anhydride group, and more preferably a group represented by the general formula: -COOR. The number of carbon atoms of each of the alkyl group and the hydroxyalkyl group is preferably 1 to 16, more preferably 1 to 6, and further preferably 1 to 3. Specific examples of the group represented by the general formula: -COOR include -COOCH₂CH₂OH, - COOCH₂CH(CH₃)OH, -COOCH(CH₃)CH₂OH, -COOH, -COOCH₃, and -COOC₂H₅. When the group represented by the general formula: -COOR is -COOH or includes -COOH, in some embodiments, -COOH is a carboxylate such as a metal carboxylate or ammonium carboxylate.

In addition, in some embodiments, the carbonyl group-containing group may be a group represented by the general formula: -X-COOR wherein X is an atomic group having a main chain composed of 1 to 20 atoms and having a molecular weight of 500 or less and R represents a hydrogen atom, an alkyl group, or a hydroxyalkyl group. The number of carbon atoms of each of the alkyl group and the hydroxyalkyl group is preferably 1 to 16, more preferably 1 to 6, and further preferably 1 to 3.

The amide group is preferably a group represented by the general formula: -CO-NRR' wherein R and R' each independently represent a hydrogen atom or a substituted or unsubstituted alkyl group, or a bond represented by the general formula: -CO-NR"-wherein R" represents a hydrogen atom, a substituted or unsubstituted alkyl group, or a substituted or unsubstituted phenyl group.

Examples of the above polar group-containing monomer include a hydroxyalkyl (meth)acrylate such as hydroxyethyl acrylate or 2-hydroxypropyl acrylate; an alkylidenemalonate ester such as dimethyl methylidenemalonate; a vinyl carboxyalkyl ether such as vinyl carboxymethyl ether or vinyl carboxyethyl ether; a carboxyalkyl (meth)acrylate such as 2-carboxyethyl acrylate or 2-carboxyethyl methacrylate; a (meth)acryloyloxyalkyl dicarboxylate ester such as acryloyloxyethyl succinate, acryloyloxypropyl succinate, methacryloyloxyethyl succinate, acryloyloxyethyl phthalate, or methacryloyloxyethyl phthalate; a monoester of an unsaturated dibasic acid such as monomethyl maleate, monoethyl maleate, monomethyl citraconate, or monoethyl citraconate; a monomer (4) represented by a general formula (4): 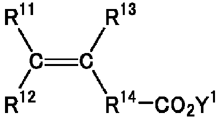 wherein R¹¹ to R¹³ each independently represent a hydrogen atom or a hydrocarbon group having 1 to 8 carbon atoms, R¹⁴ represents a single bond or a hydrocarbon group having 1 to 8 carbon atoms, and Y¹ represents an inorganic cation and/or an organic cation; an unsaturated dibasic acid such as maleic acid, maleic anhydride, citraconic acid, or citraconic anhydride.

The polar group-containing monomer unit that the fluorine-containing copolymer can contain is preferably a unit based on the monomer (4) represented by the general formula (4).

In the general formula (4), Y¹ represents an inorganic cation and/or an organic cation. Examples of the inorganic cation include a cation such as H, Li, Na, K, Mg, Ca, Al, or Fe.
Examples of the organic cation include a cation such as NH₄, NH₃R¹⁵, NH₂R¹⁵₂, NHR¹⁵₃, or NR¹⁵₄ wherein R¹⁵ independently represents an alkyl group having 1 to 4 carbon atoms. Y¹ is preferably H, Li, Na, K, Mg, Ca, Al, or NH₄, more preferably H, Li, Na, K, Mg, Al, or NH₄, further preferably H, Li, Al, or NH₄, and particularly preferably H. Specific examples of the inorganic cation and the organic cation are described by omitting their signs and valences for convenience.

In the general formula (4), R¹¹ to R¹³ each independently represent a hydrogen atom or a hydrocarbon group having 1 to 8 carbon atoms. The hydrocarbon group is a monovalent hydrocarbon group. The hydrocarbon group preferably has 4 or less carbon atoms. Examples of the hydrocarbon group include an alkyl group, an alkenyl group, and an alkynyl group having the above number of carbon atoms, and a methyl group or an ethyl group is preferable. R¹¹ and R¹² are preferably each independently a hydrogen atom, a methyl group, or an ethyl group, and R¹³ is preferably a hydrogen atom or a methyl group.

In the general formula (4), R¹⁴ represents a single bond or a hydrocarbon group having 1 to 8 carbon atoms. The hydrocarbon group is a divalent hydrocarbon group. The hydrocarbon group preferably has 4 or less carbon atoms. Examples of the hydrocarbon group include an alkylene group and an alkenylene group having the above number of carbon atoms, and among these, at least one selected from the group consisting of a methylene group, an ethylene group, an ethylidene group, a propylidene group, and an isopropylidene group is preferable, and a methylene group is more preferable.

The monomer (4) is preferably at least one selected from the group consisting of (meth)acrylic acid and a salt thereof, vinylacetic acid (3-butenoic acid) and a salt thereof, 3-pentenoic acid and a salt thereof, 4-pentenoic acid and a salt thereof, 3-hexenoic acid and a salt thereof, 4-heptenoic acid and a salt thereof, and 5-hexenoic acid and a salt thereof.

The other monomer is preferably at least one selected from the group consisting of TFE, CTFE, (meth)acrylic acid, 2,3,3,3-tetrafluoropropene, HFP, and fluoroalkyl vinyl ether.

### (Meth)acrylic acid includes acrylic acid and methacrylic acid.

The content of the other monomer unit of the fluorine-containing copolymer is preferably 0.0001 to 50.0 mol%, more preferably 0.01 mol% or more, further preferably 0.10 mol% or more, and more preferably 45.0 mol% or less, further preferably 40.0 mol% or less, particularly preferably 35.0 mol% or less, based on all monomer units.

The content of the VdF unit of the fluorine-containing copolymer is 50.0 to 99.9999 mol%, more preferably 55.0 mol% or more, further preferably 60.0 mol% or more, particularly preferably 65.0 mol% or more, and more preferably 99.99 mol% or less, further preferably 99.90 mol% or less, based on all monomer units.

When the fluorine-containing copolymer contains the fluorinated monomer unit as the other monomer unit, the content of the fluorinated monomer unit is preferably 0.0001 to 50.0 mol%, more preferably 2.0 mol% or more, further preferably 3.0 mol% or more, particularly preferably 4.0 mol% or more, and more preferably 45.0 mol% or less, further preferably 40.0 mol% or less, particularly preferably 35.0 mol% or less, based on all monomer units.

When the fluorine-containing copolymer contains the fluorinated monomer unit as the other monomer unit, the content of the VdF unit of the fluorine-containing copolymer is 50.0 to 99.999 mol%, more preferably 55.0 mol% or more, further preferably 60.0 mol% or more, particularly preferably 65.0 mol% or more, and more preferably 98.0 mol% or less, further preferably 97.0 mol% or less, particularly preferably 96.0 mol% or less, based on all monomer units.

When the fluorine-containing copolymer contains the non-fluorinated monomer unit such as the polar group-containing monomer as the other monomer unit, the content of the non-fluorinated monomer unit is preferably 0.0001 to 50.0 mol%, more preferably 0.01 mol% or more, further preferably 0.10 mol% or more, and more preferably 5.0 mol% or less, further preferably 3.0 mol% or less, particularly preferably 1.5 mol% or less, based on all monomer units.

When the fluorine-containing copolymer contains the non-fluorinated monomer unit as the other monomer unit, the content of the VdF unit of the fluorine-containing copolymer is preferably 50.0 to 99.999 mol%, more preferably 95.0 mol% or more, further preferably 97.0 mol% or more, particularly preferably 98.5 mol% or more, and more preferably 99.99 mol% or less, further preferably 99.90 mol% or less, based on all monomer units.

In the present disclosure, the formulation of the fluorine-containing copolymer can be measured, for example, by ¹⁹F-NMR measurement. In addition, when the fluorine-containing copolymer contains the polar group-containing monomer unit as the other monomer unit, the content of the polar group-containing monomer unit can be measured, for example, when the polar group is an acid group such as a carboxylic acid, by acid-base titration of the acid group.

The weight average molecular weight, in terms of polystyrene, of the fluorine-containing copolymer is preferably 10,000 to 3,000,000, more preferably 30,000 or more, further preferably 50,000 or more, particularly preferably 200,000 or more, and more preferably 2,400,000 or less, further preferably 2,200,000 or less, particularly preferably 2,000,000 or less. The weight average molecular weight can be measured by gel permeation chromatography (GPC) using dimethylformamide as a solvent.

The number average molecular weight, in terms of polystyrene, of the fluorine-containing copolymer is preferably 7,000 to 1,500,000, more preferably 21,000 or more, further preferably 35,000 or more, and more preferably 1,400,000 or less, further preferably 1,200,000 or less, particularly preferably 1,100,000 or less. The number average molecular weight can be measured by gel permeation chromatography (GPC) using dimethylformamide as a solvent.

Examples of the fluorine-containing copolymer include a VdF/(meth)acrylic acid copolymer, a VdF/TFE copolymer, a VdF/HFP copolymer, a VdF/fluoroalkyl vinyl ether copolymer, a VdF/TFE/HFP copolymer, a VdF/2,3,3,3-tetrafluoropropene copolymer, a VdF/TFE/2,3,3,3-tetrafluoropropene copolymer, a VdF/TFE/(meth)acrylic acid copolymer, a VdF/HFP/(meth)acrylic acid copolymer, a VdF/CTFE copolymer, a VdF/TFE/4-pentenoic acid copolymer, a VdF/TFE/3-butenoic acid copolymer, a VdF/TFE/HFP/(meth)acrylic acid copolymer, a VdF/TFE/HFP/4-pentenoic acid copolymer, a VdF/TFE/HFP/3-butenoic acid copolymer, a VdF/TFE/2-carboxyethyl acrylate copolymer, a VdF/TFE/HFP/2-carboxyethyl acrylate copolymer, a VdF/TFE/acryloyloxyethyl succinate copolymer, and a VdF/TFE/HFP/acryloyloxyethyl succinate copolymer.

The fluorine-containing copolymer is, among these, preferably at least one selected from the group consisting of a VdF/TFE copolymer, a VdF/CTFE copolymer, a VdF/(meth)acrylic acid copolymer, a VdF/2,3,3,3-tetrafluoropropene copolymer, a VdF/HFP copolymer, and a VdF/fluoroalkyl vinyl ether copolymer.

The VdF/TFE copolymer contains a VdF unit and a TFE unit. Use of the VdF/TFE copolymer as the copolymer allows the composition to be extremely easily formed, provides the composition having extremely excellent slurry stability, and allows the coating layer having extremely excellent flexibility to be formed. The content of the VdF unit is preferably 50.0 to 95.0 mol%, more preferably 55.0 mol% or more, further preferably 60.0 mol% or more, and more preferably 92.0 mol% or less, further preferably 89.0 mol% or less, based on all monomer units of the VdF/TFE copolymer. The content of the TFE unit is preferably 50.0 to 5.0 mol%, more preferably 45.0 mol% or less, further preferably 40.0 mol% or less, and more preferably 8.0 mol% or more, further preferably 11.0 mol% or more, based on all monomer units of the VdF/TFE copolymer.

The VdF/TFE copolymer may include a unit based on a monomer copolymerizable with VdF and TFE, excluding VdF and TFE, in addition to the VdF unit and the TFE unit. The content of the unit based on the monomer copolymerizable with VdF and TFE is preferably 3.0 mol% or less, based on all monomer units of the VdF/TFE copolymer from the viewpoint of electrolytic solution swelling resistance.

Examples of the monomer copolymerizable with VdF and TFE include the above fluorinated monomers and the above non-fluorinated monomers. The monomer copolymerizable with VdF and TFE is, among these, preferably at least one selected from the group consisting of the fluorinated monomers and the polar group-containing monomers, and more preferably at least one selected from the group consisting of HFP, 2,3,3,3-tetrafluoropropene, and the monomer (4).

The weight average molecular weight, in terms of polystyrene, of the VdF/TFE copolymer is preferably 50,000 to 2,000,000, more preferably 80,000 to 1,700,000, and further preferably 100,000 to 1,500,000.

The number average molecular weight, in terms of polystyrene, of the VdF/TFE copolymer is 35,000 to 1,400,000, more preferably 40,000 to 1,300,000, and further preferably 50,000 to 1,200,000.

The VdF/CTFE copolymer contains a VdF unit and a CTFE unit. Use of the VdF/CTFE copolymer as the copolymer allows the composition to be extremely easily formed, and allows the coating layer extremely strongly adhering to the metal foil to be formed. The content of the VdF unit is preferably 80.0 to 98.0 mol%, more preferably 85.0 mol% or more, further preferably 90.0 mol% or more, and more preferably 97.5 mol% or less, further preferably 97.0 mol% or less, based on all monomer units of the VdF/CTFE copolymer. The content of the CTFE unit is preferably 20.0 to 2.0 mol%, more preferably 15.0 mol% or less, further preferably 10.0 mol% or less, and more preferably 2.5 mol% or more, further preferably 3.0 mol% or more, based on all monomer units of the VdF/CTFE copolymer.

The VdF/CTFE copolymer may include a unit based on a monomer that can be copolymerized with VdF and CTFE, excluding VdF and CTFE, in addition to the VdF unit and the CTFE unit. The content of the unit based on the monomer that can be copolymerized with VdF and TFE is preferably 3.0 mol% or less, based on all monomer units of the VdF/CTFE copolymer from the viewpoint of electrolytic solution swelling resistance.

Examples of the monomer copolymerizable with VdF and CTFE include the above fluorinated monomers and the above non-fluorinated monomers. The monomer copolymerizable with VdF and CTFE is, among these, preferably at least one selected from the group consisting of the fluorinated monomers and the polar group-containing monomers, more preferably at least one selected from the group consisting of TFE, HFP, 2,3,3,3-tetrafluoropropene, and the monomer (4), and further preferably TFE.

The weight average molecular weight, in terms of polystyrene, of the VdF/CTFE copolymer is preferably 50,000 to 2,000,000, more preferably 80,000 to 1,700,000, and further preferably 100,000 to 1,500,000.

The number average molecular weight, in terms of polystyrene, of the VdF/CTFE copolymer is preferably 35,000 to 1,400,000, more preferably 40,000 to 1,300,000, and further preferably 50,000 to 1,200,000.

The VdF/(meth)acrylic acid copolymer contains a VdF unit and a (meth)acrylic acid unit. Use of the VdF/(meth)acrylic acid copolymer as the copolymer allows the composition to be extremely easily formed, and allows the coating layer extremely strongly adhering to the metal foil to be formed. The content of the (meth)acrylic acid unit is preferably 0.0001 to 5.0 mol%, more preferably 0.01 to 3.0 mol%, and further preferably 0.10 to 1.5 mol%, based on all monomer units.

The content of the Vdf unit of the VdF/(meth)acrylic acid copolymer is preferably 95.0 to 99.9999 mol%, more preferably 97.0 to 99.99 mol%, and further preferably 98.5 to 99.90 mol%, based on all monomer units.

The weight average molecular weight, in terms of polystyrene, of the VdF/(meth)acrylic acid copolymer is preferably 50,000 to 3,000,000, more preferably 80,000 or more, further preferably 100,000 or more, particularly preferably 200,000 or more, and more preferably 2,400,000 or less, further preferably 2,200,000 or less, particularly preferably 2,000,000 or less.

The number average molecular weight, in terms of polystyrene, of the VdF/(meth)acrylic acid copolymer is preferably 20,000 to 1,500,000, more preferably 40,000 or more, further preferably 70,000 or more, particularly preferably 140,000 or more, and more preferably 1,400,000 or less, further preferably 1,200,000 or less, particularly preferably 1,100,000 or less.

The VdF/2,3,3,3-tetrafluoropropene copolymer contains a VdF unit and a 2,3,3,3-tetrafluoropropene unit. Use of the VdF/2,3,3,3-tetrafluoropropene copolymer as the copolymer allows the composition to be extremely easily formed, and allows the coating layer having extremely excellent flexibility and electric conductivity to be formed. The content of the VdF unit is preferably 50.0 to 98.0 mol%, more preferably 55.0 mol% or more, further preferably 60.0 mol% or more, particularly preferably 65.0 mol% or more, and more preferably 97.0 mol% or less, further preferably 96.0 mol% or less, based on all monomer units of the VdF/2,3,3,3-tetrafluoropropene copolymer. The content of the 2,3,3,3-tetrafluoropropene unit is preferably 2.0 to 50.0 mol%, more preferably 3.0 mol% or more, further preferably 4.0 mol% or more, and more preferably 45.0 mol% or less, further preferably 40.0 mol% or less, particularly preferably 35.0 mol% or less, based on all monomer units of the VdF/TFE copolymer.

The VdF/2,3,3,3-tetrafluoropropene copolymer may include a unit based on a monomer that can be copolymerized with VdF and 2,3,3,3-tetrafluoropropene, excluding VdF and 2,3,3,3-tetrafluoropropene, in addition to the VdF unit and the 2,3,3,3-tetrafluoropropene unit. The content of the unit based on the monomer that can be copolymerized with VdF and 2,3,3,3-tetrafluoropropene is preferably 3.0 mol% or less, based on all monomer units of the VdF/2,3,3,3-tetrafluoropropene copolymer from the viewpoint of electrolytic solution swelling resistance.

Examples of the monomer that can be copolymerized with VdF and 2,3,3,3-tetrafluoropropene include the above fluorinated monomers and the above non-fluorinated monomers. The monomer that can be copolymerized with VdF and 2,3,3,3-tetrafluoropropene is, among these, preferably at least one selected from the group consisting of the fluorinated monomers and the polar group-containing monomers, and more preferably at least one selected from the group consisting of TFE, HFP, and the monomer (4).

The weight average molecular weight, in terms of polystyrene, of the VdF/2,3,3,3-tetrafluoropropene copolymer is preferably 10,000 to 2,000,000, more preferably 30,000 to 1,700,000, and further preferably 5,000 to 1,500,000.

The number average molecular weight, in terms of polystyrene, of the VdF/2,3,3,3-tetrafluoropropene copolymer is preferably 7,000 to 1,400,000, more preferably 21,000 to 1,300,000, and further preferably 35,000 to 1,200,000.

The VdF/HFP copolymer contains a VdF unit and a HFP unit. Use of the VdF/HFP copolymer as the copolymer allows the composition to be extremely easily formed, and allows the coating layer extremely strongly adhering to the metal foil to be formed. The content of the VdF unit is preferably 80.0 to 98.0 mol%, more preferably 83.0 mol% or more, further preferably 85.0 mol% or more, and more preferably 97.0 mol% or less, further preferably 96.0 mol% or less, based on all monomer units of the VdF/HFP copolymer. The content of the HFP unit is preferably 20.0 to 2.0 mol%, more preferably 17.0 mol% or less, further preferably 15.0 mol% or less, and more preferably 3.0 mol% or more, further preferably 4.0 mol% or less, based on all monomer units of the VdF/HFP copolymer.

The VdF/HFP copolymer may include a unit based on a monomer copolymerizable with VdF and HFP, excluding VdF and HFP, in addition to the VdF unit and the HFP unit. The content of the unit based on the monomer copolymerizable with VdF and HFP is preferably 3.0 mol% or less, based on all monomer units of the VdF/HFP copolymer from the viewpoint of electrolytic solution swelling resistance.

Examples of the monomer copolymerizable with VdF and HFP include the above fluorinated monomers and the above non-fluorinated monomers. The monomer copolymerizable with VdF and HFP is, among these, preferably at least one selected from the group consisting of the fluorinated monomers and the polar group-containing monomers, more preferably at least one selected from the group consisting of TFE, 2,3,3,3-tetrafluoropropene, and the monomer (4), and further preferably the monomer (4).

The weight average molecular weight, in terms of polystyrene, of the VdF/HFP copolymer is preferably 50,000 to 2,000,000, more preferably 80,000 to 1,700,000, and further preferably 100,000 to 1,500,000.

The number average molecular weight, in terms of polystyrene, of the VdF/HFP copolymer is preferably 35,000 to 1,400,000, more preferably 40,000 to 1,300,000, and further preferably 50,000 to 1,200,000.

The VdF/fluoroalkyl vinyl ether copolymer contains a VdF unit and a fluoroalkyl vinyl ether unit. The content of the VdF unit is preferably 80.0 to 98.0 mol%, more preferably 83.0 mol% or more, further preferably 85.0 mol% or more, and more preferably 97.0 mol% or less, further preferably 96.0 mol% or less, based on all monomer units of the VdF/fluoroalkyl vinyl ether copolymer. The content of the fluoroalkyl vinyl ether unit is preferably 20.0 to 2.0 mol%, more preferably 17.0 mol% or less, further preferably 15.0 mol% or less, and more preferably 3.0 mol% or more, further preferably 4.0 mol% or more, based on all monomer units of the VdF/fluoroalkyl vinyl ether copolymer.

The VdF/fluoroalkyl vinyl ether copolymer may include a unit based on a monomer copolymerizable with VdF and fluoroalkyl vinyl ether, excluding VdF and fluoroalkyl vinyl ether, in addition to the VdF unit and the fluoroalkyl vinyl ether unit. The content of the unit based on the monomer copolymerizable with VdF and fluoroalkyl vinyl ether is preferably 3.0 mol% or less, based on all monomer units of the VdF/fluoroalkyl vinyl ether copolymer from the viewpoint of electrolytic solution swelling resistance.

Examples of the monomer copolymerizable with VdF and fluoroalkyl vinyl ether include the above fluorinated monomers and the above non-fluorinated monomers. The monomer copolymerizable with VdF and fluoroalkyl vinyl ether is, among these, preferably at least one selected from the group consisting of the fluorinated monomers and the polar group-containing monomers, more preferably at least one selected from the group consisting of TFE, 2,3,3,3-tetrafluoropropene, and the monomer (4), and further preferably the monomer (4).

The weight average molecular weight, in terms of polystyrene, of the VdF/fluoroalkyl vinyl ether copolymer is preferably 50,000 to 2,000,000, more preferably 80,000 to 1,700,000, and further preferably 100,000 to 1,500,000.

The number average molecular weight, in terms of polystyrene, of the VdF/fluoroalkyl vinyl ether copolymer is preferably 35,000 to 1,400,000, more preferably 40,000 to 1,300,000, and further preferably 50,000 to 1,200,000.

The binder also preferably contains PVdF. The PVdF is a polymer containing a VdF unit, and is a polymer different from the above fluorine-containing copolymer. The PVdF may be a VdF homopolymer consisting only of a VdF unit in some embodiments, and may be a polymer containing a VdF unit and a unit based on a monomer copolymerizable with VdF in other embodiments.

In the PVdF, the monomer copolymerizable with VdF is preferably a monomer different from tetrafluoroethylene (TFE). That is, the PVdF preferably does not contain the TFE unit.

In the PVdF, examples of the monomer copolymerizable with VdF include a fluorinated monomer and a non-fluorinated monomer, and a fluorinated monomer is preferable. Examples of the fluorinated monomer include vinyl fluoride, trifluoroethylene, chlorotrifluoroethylene (CTFE), fluoroalkyl vinyl ether, hexafluoropropylene (HFP), (perfluoroalkyl)ethylene, 2,3,3,3-tetrafluoropropene, and trans-1,3,3,3-tetrafluoropropene. Examples of the non-fluorinated monomer include ethylene and propylene.

In the PVdF, the monomer copolymerizable with VdF is preferably at least one fluorinated monomer selected from the group consisting of CTFE, fluoroalkyl vinyl ether, HFP, and 2,3,3,3-tetrafluoropropene, and more preferably at least one fluorinated monomer selected from the group consisting of CTFE, HFP, and fluoroalkyl vinyl ether.

In the PVdF, the content of the monomer unit copolymerizable with VdF is preferably 0 to 5.0 mol%, and more preferably 0 to 3.0 mol%, based on all monomer units. In the PVdF, the content of the fluorinated monomer unit copolymerizable with VdF is preferably less than 5.0 mol%, more preferably less than 3.0 mol%, and further preferably less than 1.0 mol%, based on all monomer units.

In the present disclosure, the formulation of a PVdF can be measured, for example, by ¹⁹F-NMR measurement.

The PVdF may have a polar group. The polar group is not limited as long as it is a functional group having polarity, and is preferably at least one selected from the group consisting of a carbonyl group-containing group, an epoxy group, a hydroxy group, a sulfonic acid group, a sulfuric acid group, a phosphoric acid group, an amino group, an amide group, and an alkoxy group, more preferably at least one selected from the group consisting of a carbonyl group-containing group, an epoxy group, and a hydroxy group, and further preferably a carbonyl group-containing group. The hydroxy group does not include a hydroxy group constituting a part of the carbonyl group-containing group. In addition, the amino group is a monovalent functional group obtained by removing hydrogen from ammonia or a primary or secondary amine.

The carbonyl group-containing group is a functional group having a carbonyl group (-C(=O)-). The carbonyl group-containing group is preferably a group represented by the general formula: - COOR wherein R represents a hydrogen atom, an alkyl group, or a hydroxyalkyl group, or a carboxylic anhydride group, and more preferably a group represented by the general formula: -COOR. The number of carbon atoms of each of the alkyl group and the hydroxyalkyl group is preferably 1 to 16, more preferably 1 to 6, and further preferably 1 to 3. Specific examples of the group represented by the general formula: -COOR include -COOCH₂CH₂OH, - COOCH₂CH(CH₃)OH, -COOCH(CH₃)CH₂OH, -COOH, -COOCH₃, and -COOC₂H₅. When the group represented by the general formula: -COOR is -COOH or includes -COOH, in some embodiments, -COOH may be a carboxylate such as a metal carboxylate or ammonium carboxylate.

In addition, in some embodiments, the carbonyl group-containing group may be a group represented by the general formula: -X-COOR wherein X is an atomic group having a main chain composed of 2 to 15 atoms, and the molecular weight of the atomic group represented by X is preferably 350 or less and R represents a hydrogen atom, an alkyl group, or a hydroxyalkyl group. R represents a hydrogen atom, an alkyl group, or a hydroxyalkyl group. The number of carbon atoms of each of the alkyl group and the hydroxyalkyl group is preferably 1 to 16, more preferably 1 to 6, and further preferably 1 to 3.

The amide group is preferably a group represented by the general formula: -CO-NRR' wherein R and R' each independently represent a hydrogen atom or a substituted or unsubstituted alkyl group, or a bond represented by the general formula: -CO-NR"-wherein R" represents a hydrogen atom, a substituted or unsubstituted alkyl group, or a substituted or unsubstituted phenyl group.

The polar group can be introduced into a PVdF by polymerizing VdF and a monomer having the polar group (hereinafter, referred to as a polar group-containing monomer), or can be introduced into a PVdF by reacting the PVdF with a compound having the polar group, and it is preferable to polymerize VdF and the polar group-containing monomer from the viewpoint of productivity.

By polymerizing VdF and the polar group-containing monomer, a PVdF containing a VdF unit and a polar group-containing monomer unit is obtained. That is, the PVdF preferably contains the polar group-containing monomer unit. The content of the polar group-containing monomer unit is preferably 0.001 to 5.0 mol%, more preferably 0.01 to 3.0 mol%, and further preferably 0.10 to 1.5 mol%, based on all monomer units.

In the present disclosure, the content of the polar group-containing monomer unit in the PVdF, for example wherein the polar group is an acid group such as a carboxylic acid, can be measured by acid-base titration of the acid group.

Examples of the polar group-containing monomer include a hydroxyalkyl (meth)acrylate such as hydroxyethyl acrylate or 2-hydroxypropyl acrylate; an unsaturated monobasic acid such as (meth)acrylic acid, crotonic acid, vinylacetic acid (3-butenoic acid), 3-pentenoic acid, 4-pentenoic acid, 3-hexenoic acid, or 4-heptenoic acid; an unsaturated dibasic acid such as maleic acid, maleic anhydride, citraconic acid, or citraconic anhydride; an alkylidenemalonate ester such as dimethyl methylidenemalonate; a vinyl carboxyalkyl ether such as vinyl carboxymethyl ether or vinyl carboxyethyl ether; a carboxyalkyl (meth)acrylate such as 2-carboxyethyl acrylate or 2-carboxyethyl methacrylate; a (meth)acryloyloxyalkyl dicarboxylate ester such as acryloyloxyethyl succinate, methacryloyloxyethyl succinate, acryloyloxyethyl phthalate, acryloyloxypropyl succinate, or methacryloyloxyethyl phthalate; and a monoester of an unsaturated dibasic acid such as monomethyl maleate, monoethyl maleate, monomethyl citraconate, or monoethyl citraconate.

When a PVdF is reacted with a compound having the polar group to introduce the polar group into the PVdF, the compound having the polar group may be the polar group-containing monomer, or a silane-based coupling agent or a titanate-based coupling agent having a group that is reactive with the PVdF and a hydrolyzable group. The hydrolyzable group is preferably an alkoxy group. When a coupling agent is used, the coupling agent can be reacted with a PVdF dissolved or swollen in a solvent to add the polar group to the PVdF.

The PVdF may also be obtained by partially dehydrofluorinating a PVdF using a base and then further reacting the partially dehydrofluorinated PVdF with an oxidizing agent. Examples of the oxidizing agent include hydrogen peroxide, a hypochlorite, palladium halide, chromium halide, an alkali metal permanganate, a peracid compound, an alkyl peroxide, and an alkyl persulfate.

The content of the VdF unit of the PVdF is preferably more than 95.0 mol%, more preferably more than 97.0 mol%, and further preferably more than 99.0 mol%, based on all monomer units.

In addition, the content of the VdF unit of the PVdF is preferably 95.0 to 99.999 mol%, more preferably 97.0 mol% or more, more preferably 98.5 mol% or more, and more preferably 99.99 mol% or less, more preferably 99.90 mol% or less, based on all monomer units.

The weight average molecular weight, in terms of polystyrene, of the PVdF is preferably 50,000 to 3,000,000, more preferably 80,000 or more, further preferably 100,000 or more, particularly preferably 200,000 or more, and more preferably 2,400,000 or less, further preferably 2,200,000 or less, particularly preferably 2,000,000 or less. The weight average molecular weight can be measured by gel permeation chromatography (GPC) using N,N-dimethylformamide as a solvent. In addition, the weight average molecular weight of the PVdF may be 1,000,000 or more in some embodiments, and may be 1,500,000 or more in other embodiments.

The number average molecular weight, in terms of polystyrene, of the PVdF is preferably 20,000 to 1,500,000, more preferably 40,000 or more, further preferably 70,000 or more, particularly preferably 140,000 or more, and more preferably 1,400,000 or less, further preferably 1,200,000 or less, particularly preferably 1,100,000 or less. The number average molecular weight can be measured by gel permeation chromatography (GPC) using dimethylformamide as a solvent.

The melting point of the PVdF is preferably 100 to 240°C. The melting point can be determined as the temperature corresponding to the maximum value on the heat of fusion curve when the temperature is raised at a rate of 10°C/min by using a differential scanning calorimetry (DSC) apparatus.

The binder also preferably contains PVdF and a fluorine-containing copolymer containing a VdF unit and a unit of an other monomer other than VdF, as the fluorine-containing polymer.

When the binder contains the PVdF and the fluoroine-containing copolymer, the mass ratio of the PVdF to the fluorine-containing copolymer in the binder is preferably 99/1 to 1/99, more preferably 97/3 to 3/97, further preferably 95/5 to 5/95, still further preferably 90/10 to 10/90, particularly preferably 85/15 to 15/85, and most preferably 80/20 to 40/60.

The binder may contain an other polymer, in addition to the PVdF and the fluorine-containing copolymer. Examples of the other polymer include polymethacrylate, polymethyl methacrylate, polyacrylonitrile, polyimide, polyamide, polyamideimide, polycarbonate, styrene elastomer, and butadiene elastomer.

The content of the fluorine-containing copolymer in the binder is preferably 1% by mass or more, more preferably 3% by mass or more, further preferably 5% by mass or more, particularly preferably 10% by mass or more, most preferably 15% by mass or more, and may be 100% by mass or less in some embodiments, based on the mass of the binder.

The content of the binder in the composition of the present disclosure is preferably 0.1 to 20% by mass, more preferably 0.2 to 10% by mass, and further preferably 0.5 to 3% by mass, based on the mass of the composition.

The viscosity of the composition of the present disclosure is preferably 5 to 8,000 mPa·s, more preferably 100 to 5,000 mPa·s, and further preferably 100 to 2,000 mPa·s. The viscosity can be measured under conditions of 25°C, and rotor No. SC4-21, and a rotation speed of 20 rpm by using a B-type viscometer (LV-DV2T, manufactured by Brookfield).

### (Use)

The composition of the present disclosure can be suitably used to form an electrode of an electrochemical device. In some embodiments, the composition of the present disclosure may be a composition for electrode formation.

The composition of the present disclosure can be suitably used as a material for forming an electrode of a battery such as a secondary battery or a capacitor. The battery may be a primary battery in some embodiments, and may be a storage battery (secondary battery) or an energy storage element in other embodiments. In some embodiments, the battery may be a non-aqueous electrolytic solution battery. The non-aqueous electrolytic solution battery includes all batteries including an electrolytic solution and a power generation element. Examples of the non-aqueous electrolytic solution battery include a lithium ion primary battery, a lithium ion secondary battery, a nickel hydrogen battery, a lithium ion capacitor, an electric double layer capacitor, and a sodium ion secondary battery.

The composition of the present disclosure may be a composition for positive electrode formation used to create a positive electrode in some embodiments, and may be a composition for negative electrode formation used to create a negative electrode in other embodiments. The electrode material layer formed from the composition of the present disclosure may be a positive electrode material layer in some embodiments, and may be a negative electrode material layer in other embodiments.

### (Other component)

The composition of the present disclosure preferably further contains a powder electrode material, excluding the single-walled carbon nanotube.

The powder electrode material is a powder electrode material used for a battery, and preferably includes an electrode active material. The electrode active material is divided into a positive electrode active material and a negative electrode active material.

The positive electrode active material may be a material that can electrochemically absorb and desorb a lithium ion, and is preferably a lithium composite oxide, and more preferably a lithium transition metal composite oxide. The positive electrode active material is also preferably a lithium-containing transition metal phosphate compound. The positive electrode active material is also preferably a substance containing lithium and at least one transition metal, such as a lithium transition metal composite oxide or a lithium-containing transition metal phosphate compound.

Preferable examples of the transition metal of the lithium transition metal composite oxide include V, Ti, Cr, Mn, Fe, Co, Ni, and Cu, and specific examples of the lithium transition metal composite oxide include a lithium-cobalt composite oxide such as LiCoO₂, a lithium-nickel composite oxide such as LiNiO₂, a lithium-manganese composite oxide such as LiMnO₂, LiMn₂O₄, or Li₂MnO₃, and compounds obtained by replacement of a part of the transition metal atoms mainly constituting these lithium transition metal composite oxides with another metal such as Al, Ti, V, Cr, Mn, Fe, Co, Li, Ni, Cu, Zn, Mg, Ga, Zr, or Si. Examples of the compounds obtained by replacement include a lithium-nickel-manganese composite oxide, a lithium-nickel-cobalt-aluminum composite oxide, a lithium-nickel-cobalt-manganese composite oxide, a lithium-manganese-aluminum composite oxide, and a lithium-titanium composite oxide, and more specific examples include LiNi_{0.5}Mn_{0.5}O₂, LiNi_{0.85}Co_{0.10}Al_{0.05}O₂, LiNi_{0.33}Co_{0.33}Mn_{0.33}O₂, LiNi_{0.5}Mn_{0.3}Co_{0.2}O₂, LiNi_{0.6}Mn_{0.2}Co_{0.2}O₂, LiNi_{0.8}Mn_{0.1}Co_{0.1}O₂, LiMn_{1.8}Al_{0.2}O₄, LiMn_{1.5}Ni_{0.5}O₄, Li₄Ti₅O₁₂, and LiNi_{0.82}Co_{0.15}Al_{0.03}O₂.

Preferable examples of the transition metal of the lithium-containing transition metal phosphate compound include V, Ti, Cr, Mn, Fe, Co, Ni, and Cu, and specific examples of the lithium-containing transition metal phosphate compound include an iron phosphate compound such as LiFePO₄, Li₃Fe₂(PO₄)₃, or LiFeP₂O₇, a cobalt phosphate compound such as LiCoPO₄, and compounds obtained by replacement of a part of the transition metal atoms mainly constituting these lithium transition metal phosphate compounds with another metal such as Al, Ti, V, Cr, Mn, Fe, Co, Li, Ni, Cu, Zn, Mg, Ga, Zr, Nb, or Si.

In particular, LiCoO₂, LiNiO₂, LiMn₂O₄, LiNi_{0.82}Co_{0.15}Al_{0.03}O₂, LiNi_{0.33}Mn_{0.33}Co_{0.33}O₂, LiNi_{0.5}Mn_{0.3}Co_{0.2}O₂, LiNi_{0.6}Mn_{0.2}Co_{0.2}O₂, LiNi_{0.8}Mn_{0.1}Co_{0.1}O₂, and LiFePO₄ are preferable from the viewpoint of high voltage, high energy density, charge/discharge cycle characteristics, or the like.

In addition, the lithium transition metal composite oxide is preferably a lithium-nickel-based composite oxide, and more preferably a lithium-nickel-based composite oxide represented by the general formula (7):

general formula (7): Li_{y}Ni₁₋ₓMₓO₂

wherein x is 0.01 ≤ x ≤ 0.5, y is 0.9 ≤ y ≤ 1.2, and M represents a metal atom excluding Li and Ni. The lithium transition metal composite oxide having such a high nickel content is beneficial for increasing the capacity of a secondary battery.

In the general formula (7), x is a coefficient satisfying 0.01 ≤ x ≤ 0.5, and from the viewpoint of being able to obtain a secondary battery having a higher capacity, preferably 0.05 ≤ x ≤ 0.4 and more preferably 0.10 ≤ x ≤ 0.3.

In the general formula (7), examples of the metal atom of M include V, Ti, Cr, Mn, Fe, Co, Cu, Al, Zn, Mg, Ga, Zr, and Si. The metal atom of M is preferably a transition metal such as V, Ti, Cr, Mn, Fe, Co, or Cu, or a combination of any of the above transition metals and a further metal such as Al, Ti, V, Cr, Mn, Fe, Co, Cu, Zn, Mg, Ga, Zr, or Si.

The lithium transition metal composite oxide having a high nickel content is preferably at least one selected from the group consisting of LiNi_{0.80}Co_{0.15}Al_{0.05}O₂, LiNi_{0.82}Co_{0.15}Al_{0.03}O₂, LiNi_{0.33}Mn_{0.33}Co_{0.33}O₂, LiNi_{0.5}Mn_{0.3}Co_{0.2}O₂, LiNi_{0.6}Mn_{0.2}Co_{0.2}O₂, LiNi_{0.8}Mn_{0.1}Co_{0.1}O₂, and LiNi_{0.90}Mn_{0.05}Co_{0.05}O₂, and more preferably at least one selected from the group consisting of LiNi_{0.82}Co_{0.15}Al_{0.03}O₂, LiNi_{0.6}Mn_{0.2}Co_{0.2}O₂, and LiNi_{0.8}Mn_{0.1}Co_{0.1}O₂.

In addition, these positive electrode active materials used may each have a surface to which a substance different in formulation from the substance mainly constituting the positive electrode active material adheres. Examples of the surface adhering substance include an oxide such as aluminum oxide, silicon oxide, titanium oxide, zirconium oxide, magnesium oxide, calcium oxide, boron oxide, antimony oxide, or bismuth oxide, a sulfate such as lithium sulfate, sodium sulfate, potassium sulfate, magnesium sulfate, calcium sulfate, or aluminum sulfate, and a carbonate such as lithium carbonate, calcium carbonate, or magnesium carbonate.

These surface adhering substances can each be adhered to the surface of a positive electrode active material, for example, by a method involving dissolving or suspending the surface adhering substance in a solvent to add the resulting solution or suspension to the positive electrode active material by impregnation and drying the same, a method involving dissolving or suspending a surface adhering substance precursor in a solvent to add the resulting solution or suspension to the positive electrode active material by impregnation and then reacting these by heating or the like, or a method involving adding the surface adhering substance to a positive electrode active substance precursor and simultaneously calcining these.

The lower limit of the amount by mass of the surface adhering substance used is preferably 0.1 ppm or more, more preferably 1 ppm or more, and further preferably 10 ppm or more, and the upper limit thereof is preferably 20% or less, more preferably 10% or less, and further preferably 5% or less based on the positive electrode active material. The surface adhering substance can suppress the oxidation reaction of the non-aqueous electrolytic solution on the surface of the positive electrode active material and improve the battery life, and if the amount thereof adhered is too small, the effects thereof are not sufficiently developed, and if the amount is too large, the resistance may increase because the surface adhering substance inhibits the entry and exit of a lithium ion.

A particle of the positive electrode active material may have a blocky shape, a polyhedral shape, a spherical shape, an oval shape, a plate-like shape, a needle-like shape, a columnar shape, or the like as conventionally used, and among these, preferably, a primary particle aggregates to form a secondary particle that has a spherical shape or an oval shape. Usually, the active material in the electrode expands and contracts with the charge and discharge of an electrochemical element, and thus the stress thereof is likely to cause a deterioration such as destruction of the active material or disconnection of a conductive path. Because of this, an active material in which a primary particle aggregates to form a secondary particle is more preferable than a single particle active material containing only a primary particle because the former relaxes the stress of expansion and contraction and prevents a deterioration. In addition, a particle having a spherical shape or an oval shape is more preferable than an axially oriented particle such as a particle having a plate-like shape because the former particle is less oriented when an electrode is molded and thus the electrode also expands and contracts less during charge and discharge, and even when the former particle is mixed with a conductive agent in electrode creation, it is easy to mix the former particle uniformly.

The tap density of the positive electrode active material is usually 1.3 g/cm³ or more, preferably 1.5 g/cm³ or more, further preferably 1.6 g/cm³ or more, and most preferably 1.7 g/cm³ or more. If the tap density of the positive electrode active material is lower than the above lower limit, when forming a positive electrode material layer, the required amount of a dispersion medium increases, the required amount of a conductive agent or a copolymer increases, and the rate of filling of the positive electrode material layer with the positive electrode active material may be limited to limit the battery capacity. A high density positive electrode material layer can be formed by using a metal composite oxide powder having a high tap density. In general, a larger tap density is more preferable, and there is no particular upper limit thereon, and if the tap density is too large, the diffusion of a lithium ion using a non-aqueous electrolytic solution as a medium in the positive electrode material layer may be rate-determining to easily reduce the load characteristics, and thus the upper limit of the tap density is usually 2.5 g/cm³ or less and preferably 2.4 g/cm³ or less.

The density determined as follows is defined as the tap density of the positive electrode active material: a sample is passed through a sieve having an opening size of 300 µm, dropped into a tapping cell of 20 cm³ to fill the cell volume, then tapping is carried out at a stroke length of 10 mm 1000 times using a powder density measuring instrument (for example, Tap Denser manufactured by Seishin Enterprise Co., Ltd.), and the density is determined from the volume at that time and the weight of the sample.

The median diameter d50 (secondary particle size when a primary particle aggregates to form a secondary particle) of the particle of the positive electrode active material is usually 0.1 µm or more, preferably 0.5 µm or more, more preferably 1 µm or more, most preferably 3 µm or more, and usually 20 µm or less, preferably 18 µm or less, more preferably 16 µm or less, most preferably 15 µm or less. If d50 is lower than the above lower limit, it may not be possible to obtain a high bulk density product, and if d50 exceeds the upper limit, for example the following problems may occur: it takes time to diffuse lithium in the particles, resulting in reduction in battery performance, and when creating a positive electrode of a battery, that is, slurrying an active material and a conductive agent, a copolymer, or the like using a solvent to apply the resulting slurry in the form of a thin film, a streak is formed. Here, mixing of two or more positive electrode active materials having different median diameters d50 can also further improve the filling property when creating a positive electrode.

The median diameter d50 in the present disclosure is measured using a known laser diffraction/scattering type particle size distribution measurement apparatus. When LA-920 manufactured by HORIBA, Ltd. is used as a particle size distribution analyzer, d50 is measured by using a 0.1% by mass sodium hexametaphosphate aqueous solution as a dispersion medium used for the measurement, carrying out ultrasonic dispersion for 5 minutes, and then setting the measurement refractive index to 1.24.

When a primary particle aggregates to form a secondary particle, the average primary particle size of the positive electrode active material is usually 0.01 µm or more, preferably 0.05 µm or more, further preferably 0.08 µm or more, most preferably 0.1 µm or more, and usually 3 µm or less, preferably 2 µm or less, further preferably 1 µm or less, most preferably 0.6 µm or less. If the average primary particle size exceeds the above upper limit, it is difficult to form a spherical secondary particle, which adversely affects the powder filling property and greatly reduces the specific surface area, and thus battery performance such as output characteristics may be likely to decrease. On the contrary, if the average primary particle size is lower than the above lower limit, a crystal is usually underdeveloped, which may cause a problem such as poor charge/discharge reversibility. The primary particle size is measured by observation using a scanning electron microscope (SEM). Specifically, the primary particle size is determined by obtaining the value of the longest segment of a straight line in the horizontal direction formed by the left and right boundary lines of a primary particle on a photograph at a magnification of 10,000 times for each of any 50 primary particles and averaging the obtained values.

The BET specific surface area of the positive electrode active material is 0.2 m²/g or more, preferably 0.3 m²/g or more, further preferably 0.4 m²/g or more, and 4.0 m²/g or less, preferably 2.5 m²/g or less, further preferably 1.5 m²/g or less. If the BET specific surface area is smaller than this range, the battery performance easily decreases, and if the BET specific surface area is larger than this range, the tap density does not easily increase, and a problem may easily occur in the coating performance when forming the positive electrode material layer.

The BET specific surface area is defined as a value determined using a surface area meter (for example, a fully automatic surface area measurement apparatus manufactured by Ohkura Riken) by pre-drying a sample at 150°C for 30 minutes under a nitrogen flow and then measuring the sample by the nitrogen adsorption BET single point method according to the gas flow method using a nitrogen helium mixed gas accurately adjusted such that the value of nitrogen pressure relative to atmospheric pressure is 0.3.

The method for producing a positive electrode active material may be a general method used as a method for producing an inorganic compound. In particular, various methods can be considered for creating a spherical or oval active material, and examples thereof include a method for obtaining an active material by dissolving or pulverizing and dispersing a transition metal raw material such as a transition metal nitrate or sulfate and optionally a raw material of another element in a solvent such as water and regulating the pH while stirring to create and recover a spherical precursor, optionally drying this precursor, then adding a Li source such as LiOH, Li₂CO₃, or LiNO₃, and calcining the resulting mixture at a high temperature, a method for obtaining an active material by dissolving or pulverizing and dispersing a transition metal raw material such as a transition metal nitrate, sulfate, hydroxide, or oxide and optionally a raw material of another element in a solvent such as water, drying and molding the resulting solution or suspension using a spray dryer or the like to obtain a spherical or oval precursor, adding a Li source such as LiOH, Li₂CO₃, or LiNO₃ to this precursor, and calcining the resulting mixture at a high temperature, and a method for obtaining an active material by dissolving or pulverizing and dispersing a transition metal raw material such as a transition metal nitrate, sulfate, hydroxide, or oxide, a Li source such as LiOH, Li₂CO₃, or LiNO₃, and optionally a raw material of another element in a solvent such as water, drying and molding the resulting solution or suspension using a spray dryer or the like to obtain a spherical or oval precursor, and calcining this precursor at a high temperature.

In the present disclosure, one positive electrode active material may be used alone, or two or more positive electrode active material powders having different formulations or different powder physical characteristics may be used together in any combination and ratio.

When the composition of the present disclosure contains a positive electrode active material, the content of the single-walled carbon nanotubes is preferably 0.001 to 10 parts by mass per 100 parts by mass of the positive electrode active material.

When the composition of the present disclosure contains a positive electrode active material, the content of the binder is preferably 0.01 to 5.0 parts by mass per 100 parts by mass of the positive electrode active material.

Examples of negative electrode active material include a negative electrode active material that exhibits a potential of 2.5 V or less vs. Li when alloyed with Li or bonded with Li.

The negative electrode active material may be a negative electrode active material containing a metal. The metal included in the negative electrode active material is usually a metal that can be electrochemically alloyed with an alkali metal such as Li or Na.

Examples of the negative electrode active material include a simple metal that can be electrochemically alloyed with Li such as Si, Zn, Sn, W, Al, Sb, Ge, Bi, or In; an alloy including Si, Zn, Sn, W, Al, Sb, Ge, Bi, or In; a lithium alloy such as a lithium aluminum alloy or a lithium tin alloy; a metal oxide such as tin oxide or silicon oxide; lithium titanate. One or two or more of these negative electrode active materials can be used.

The negative electrode active material is preferably a compound containing at least one element selected from the group consisting of Si, Sn, V, Nb, and Ti, more preferably Si (simple Si), an oxide of Si, an alloy containing Si, Sn (Sn carrier), an oxide of Sn, or an alloy containing Sn, and further preferably at least one selected from the group consisting of Si and SiOₓ (0 < x < 2) .

In addition, the negative electrode active material may be a carbonaceous material such as a graphite powder. The negative electrode active material can be used together with a negative electrode active material containing a metal. Examples of the carbonaceous material include natural graphite, an artificial carbonaceous substance, an artificial graphite substance, and a carbonaceous material obtained by one or more times of heat treatment in the range of 400°C to 3,200°C of a carbonaceous substance {for example, natural graphite, coal-based coke, petroleum-based coke, coal-based pitch, petroleum-based pitch, or a material obtained by oxidation treatment of any of these pitches; needle coke, pitch coke, or a carbon material obtained by partial graphitization of any of these cokes; a pyrolysate of an organic substance such as furnace black, acetylene black, ketjen black, or pitch-based carbon fiber; a carbonizable organic substance (for example, coal tar pitches from soft pitch to hard pitch; a coal-based heavy oil such as dry distillation/liquefaction oil; a straight-run heavy oil such as atmospheric residue or vacuum residue; a cracking-derived petroleum heavy oil such as ethylene tar as a byproduct generated in thermal cracking of crude oil, naphtha, or the like; further, an aromatic hydrocarbon such as acenaphthylene, decacyclene, anthracene, or phenanthrene; a N-ring compound such as phenazine or acridine; a S-ring compound such as thiophene or bithiophene; a polyphenylene such as biphenyl or terphenyl; polyvinyl chloride, polyvinyl alcohol, polyvinyl butyral, or a material obtained by insolubilizing any thereof; a nitrogen-containing organic polymer such as polyacrylonitrile or polypyrrole; a sulfur-containing organic polymer such as polythiophene or polystyrene; a natural polymer such as a polysaccharide typified by cellulose, lignin, mannan, polygalacturonic acid, chitosan, or saccharose; a thermoplastic resin such as polyphenylene sulfide or polyphenylene oxide; a thermosetting resin such as a furfuryl alcohol resin, a phenol-formaldehyde resin, or an imide resin) and a carbide of any of these; and a solution obtained by dissolving a carbonizable organic substance in a low-molecular-weight organic solvent such as benzene, toluene, xylene, quinoline, or n-hexane, and a carbide thereof}.

When the negative electrode mixture contains a negative electrode active material containing a metal and a carbonaceous material, the mass ratio of the negative electrode active material containing a metal to the carbonaceous material is preferably 1/99 to 99/1, more preferably 5/95 to 95/5, and further preferably 10/10 to 90/10.

When the composition of the present disclosure contains a negative electrode active material, the content of the single-walled carbon nanotubes is preferably 0.001 to 10 parts by mass per 100 parts by mass of the negative electrode active material.

When the composition of the present disclosure contains a negative electrode active material, the content of the binder is preferably 0.01 to 20.0 parts by mass per 100 parts by mass of the positive electrode active material.

The content of the electrode active material (positive electrode active material or negative electrode active material) is preferably 40% by mass or more in the composition for electrode formation in order to increase the capacity of the electrode obtained.

The powder electrode material may further include a conductive agent. Examples of the conductive agent include a carbon black such as acetylene black or ketjen black, a carbon material such as graphite, a carbon fiber, a carbon nanotube, a carbon nanohorn, and graphene.

The proportion by mass of the powder electrode materials, the active materials and the conductive agent, to the above copolymer in the composition is usually approximately 80:20 to 99.5:0.5 and is determined in consideration of the holding of the powder component, the adhesion to the current collector, and the conductivity of the electrode.

The composition may contain, for example, an acrylic resin such as polyacrylic acid, polymethacrylate, or polymethyl methacrylate, a polyimide, polyamide, or polyamideimide-based resin, styrene elastomer, butadiene elastomer, or styrene-butadiene elastomer.

In order to improve the slurry stability, the composition may contain a dispersant such as a resin-based dispersant having a surfactant action and the like, a cationic surfactant, or a nonionic surfactant.

The composition can be prepared by mixing the single-walled carbon nanotube, the binder, and the solvent. A powder electrode material may be further dispersed or mixed into the obtained composition. Then, the obtained composition is uniformly applied to a current collector such as a metal foil or a metal net, dried, and optionally pressed to form a thin electrode material layer on the current collector to obtain a thin-film electrode. In addition, the composition may be prepared by first mixing single-walled carbon nanotubes, a binder, and a powder electrode material and then adding a solvent.

### (Electrode)

The electrode of the present disclosure includes a current collector and an electrode material layer. The electrode material layer is formed by using the composition of the present disclosure, and may be provided on one side of the current collector in some embodiments, and may be on each of both sides thereof in other embodiments. The electrode of the present disclosure may be a positive electrode including a positive electrode material layer in some embodiments, and may be a negative electrode having a negative electrode material layer in other embodiments.

Since the electrode of the present disclosure includes an electrode material layer formed using the composition of the present disclosure, use of the electrode of the present disclosure in a battery allows a battery having excellent output characteristics, cycle characteristics, and 60°C storage characteristics and having a reduced gas generation rate to be obtained.

The density of the electrode material layer is preferably 2.0 to 5.0 g/cm3, and more preferably 2.5 to 4.5 g/cm³.

The density of the electrode material layer can be calculated from the mass and the volume of the electrode material layer.

The thickness of the electrode material layer is preferably 20 µm or more, more preferably 30 µm or more, further preferably 40 µm or more, particularly preferably 45 µm or more, and preferably 170 µm or less, more preferably 150 µm or less, from the viewpoint of obtaining further superior battery characteristics. In addition, the thickness of the electrode material layer may be 85 µm or less in some embodiments, and may be less than 69 µm in other embodiments.

The thickness of the electrode material layer can be measured with a micrometer. The thickness of the electrode material layer in the present disclosure is the thickness per side when the electrode material layer is provided on each of both sides of the current collector.

Examples of the current collector included in the electrode of the present disclosure include a metal foil or a metal net of iron, stainless steel, copper, aluminum, nickel, titanium, or the like, and among these, aluminum foil is preferable.

The electrode of the present disclosure can be suitably produced by a production method involving applying the composition of the present disclosure to a current collector. After applying the composition, further, the coating film may be dried, and the resulting dry coating film may be pressed.

The amount of the composition applied to the current collector is preferably 10 mg/cm² or more, more preferably 17.5 mg/cm² or more, and preferably 60 mg/cm² or less, more preferably 50 mg/cm² or less. The amount of the composition applied is the dry weight of the composition per unit area.

### (Secondary battery)

In addition, according to the present disclosure, a secondary battery including the above electrode is provided.

A secondary battery of the present disclosure includes an electrode formed using the composition of the present disclosure and thus has excellent output characteristics, cycle characteristics, and 60°C storage characteristics and has a low gas generation rate.

The secondary battery of the present disclosure is preferably one including a positive electrode, a negative electrode, and a non-aqueous electrolytic solution, wherein one or both of the positive electrode and the negative electrode is/are the electrode(s) described above. In addition, the secondary battery of the present disclosure is preferably one including a positive electrode, a negative electrode, and a non-aqueous electrolytic solution, wherein the positive electrode is the electrode described above. In addition, a separator may be interposed between the positive electrode and the negative electrode.

The non-aqueous electrolytic solution is not limited, and may be one or two or more of known solvents such as propylene carbonate, ethylene carbonate, butylene carbonate, γ-butyl lactone, 1,2-dimethoxyethane, 1,2-diethoxyethane, dimethyl carbonate, diethyl carbonate, or ethyl methyl carbonate. Any conventionally known electrolyte can also be used, and LiClO₄, LiAsF₆, LiPF₆, LiBF₄, LiCl, LiBr, CH₃SO₃Li, CF₃SO₃Li, cesium carbonate, or the like can be used.

The electrode of the present disclosure has excellent flexibility and conductivity, has the current collector and the electrode material layer sufficiently adhered to each other, and can form a secondary battery having excellent battery characteristics, and thus can be suitably used as an electrode for a wound type secondary battery. In addition, in some embodiments, the secondary battery of the present disclosure may be a wound type secondary battery.

The electrode of the present disclosure is useful not only for the lithium ion secondary battery using a liquid electrolyte described above, but also for a polymer electrolyte lithium secondary battery, as an electrode for a non-aqueous electrolyte secondary battery. In addition, the electrode of the present disclosure is also useful for an electric double layer capacitor.

The embodiments have been described above, and it will be understood that various modifications of the embodiments and details are possible without departing from the spirit and scope of the claims.

### EXAMPLES

Next, the embodiments of the present disclosure will be described with reference to Examples, but the present disclosure is not limited only to the Examples.

The numerical values in the Examples were measured by the following methods.

### (Ratio between VdF unit and TFE unit in fluorine-containing copolymer)

The ratio between VdF unit and TFE unit in a fluorine-containing copolymer was measured in a DMF-d₇ solution state of a polymer by ¹⁹F-NMR measurement using an NMR analyzer (manufactured by Agilent Technologies Japan, Ltd., VNS400MHz).

In the ¹⁹F-NMR measurement, the areas (A, B, C, and D) of the following peaks were determined, and the ratio between VdF unit and TFE unit was calculated.
A: Area of a peak of -86 ppm to -98 ppm
B: Area of a peak of -105 ppm to -118 ppm
C: Area of a peak of -119 ppm to -122 ppm
D: Area of a peak of -122 ppm to -126 ppm

Proportion of VdF unit: (4A + 2B)/(4A + 3B + 2C + 2D) × 100 [mol%]
Proportion of TFE unit: (B + 2C + 2D)/(4A + 3B + 2C + 2D) × 100 [mol%]

### (Weight average molecular weight)

The weight average molecular weight was measured by gel permeation chromatography (GPC). The weight average molecular weight was calculated from data (reference: polystyrene) obtained by measurement using HLC-8320GPC and columns (three Super AWM-H connected in series) manufactured by Tosoh Corporation under a flow of dimethylformamide (DMF) as a solvent at a flow rate of 0.3 ml/min.

### (Melting point)

The melting point was determined as the temperature corresponding to the maximum value in the heat of fusion curve when the temperature was raised from 30°C to 220°C at a rate of 10°C/min, then lowered to 30°C at 10°C/min, and raised again to 220°C at a rate of 10°C/min by using a differential scanning calorimetry (DSC) apparatus.

### (Viscosity)

The viscosity of the composition was measured under conditions of 25°C, rotor No. SC4-21, and a rotation speed of 20 rpm by using a B-type viscometer (LV-DV2T, manufactured by Brookfield), and the measured value 10 minutes after the start of measurement was defined as the viscosity.

In the examples and comparative examples, polymers having the following physical properties were used as binders.
Fluorine-containing copolymer (A): Fluorine-containing copolymer containing VdF unit and TFE unit
VdF/TFE = 81/19 (mol%)
Weight average molecular weight: 1,230,000
Melting point 128°C
PVdF (B): VdF homopolymer
Weight average molecular weight: 1,800,000
Melting point 171°C

In addition, in the examples and the comparative examples, the following positive electrode active materials were used.
NMC811: LiNi_{0.8}Mn_{0.1}Co_{0.1}O₂

In addition, in the examples and the comparative examples, the following single-walled carbon nanotubes were used.

### Single-walled carbon nanotube (trade name "TUBALL BATT SWCNT", manufactured by OCSiAl)

Average outer diameter: 1.6 ± 0.4 nm
Length: 5 µm or more
Average G/D ratio: 86.5 ± 7.1

### Example 1

### (Preparation of composition)

The single-walled carbon nanotube, the fluorine-containing copolymer (A), and 3-methoxy-N,N-dimethylpropaneamide were mixed to prepare a composition having a formulation shown in Table 1. The viscosity of the obtained composition was measured. The result is shown in Table 1.

### (Preparation of positive electrode mixture)

The obtained composition and PVdF (B) were mixed so that the mass ratio of the fluorine-containing copolymer (A) to PVdF (B) became as shown in Table 1. The obtained mixed solution, NMC811, acetylene black, and 3-methoxy-N,N-dimethylpropaneamide were mixed to prepare a positive electrode mixture having a solid content concentration of 71% by mass. The formulation of the obtained positive electrode mixture is shown in Table 1.

### (Creation of positive electrode)

The obtained positive electrode mixture was uniformly applied to one side of a positive electrode current collector (aluminum foil having a thickness of 20 µm) and dried at 120°C for 60 minutes to completely volatilize 3-methoxy-N,N-dimethylpropaneamide, and then the positive electrode current collector was pressed by applying a pressure of 10 tons by using a roll press, to create a positive electrode including a positive electrode material layer and the positive electrode current collector.

### (Creation of negative electrode)

An artificial graphite powder as a negative electrode active material, an aqueous emulsion of carboxylmethyl cellulose sodium (concentration of sodium carboxylmethyl cellulose of 1% by mass) as a thickener, and an aqueous dispersion of styrene-butadiene elastomer (concentration of styrene-butadiene elastomer of 50% by mass) as a binder were used and mixed in an aqueous solvent in a solid content ratio of the active material, the thickener, and the binder of 97.6/1.2/1.2 (% by mass ratio) to prepare a negative electrode mixture slurry in a slurry form. The obtained negative electrode mixture slurry was uniformly applied to a copper foil having a thickness of 20 µm, dried, and compression-molded to create a negative electrode.

### (Creation of lithium ion secondary battery)

The positive electrode, the negative electrode, and a separator made of polyethylene were laminated in the order of the negative electrode, the separator, and the positive electrode to create a battery element.

This battery element was intercalated into a bag made of a laminate film, which was an aluminum sheet (having a thickness of 40 µm) covered with resin layers on both sides, while the terminals of the positive electrode and the negative electrode were provided to protrude. Next, 1.2 g of an electrolytic solution (an electrolytic solution obtained by dissolving LiPF₆ at a concentration of 1 mol/liter in a solvent obtained by mixing ethylene carbonate and ethyl methyl carbonate in a volume ratio of 3/7 and further adding 2% by mass of vinylene carbonate thereto) was each injected into the bag, and vacuum sealing was performed, to create a sheet-like lithium ion secondary battery.

### (Output characteristics)

At 25°C, the lithium ion secondary battery created above was constant-current constant-voltage-charged (hereinafter, referred to as CC/CV charging) to 4.2 V at a constant current corresponding to 0.2 C (0.1 C cut), and then discharged to 3 V at a constant current of 0.2 C. This was regarded as 1 cycle, and the initial discharge capacity was determined from the discharge capacity at the 3^{rd} cycle. Here, 1.0 C represents a current value for discharging the reference capacity of a battery in 1 hour, and for example, 0.2 C represents a current value equal to 1/5 thereof. The battery for which the evaluation of the initial discharge capacity had been ended was charged at a constant current of 0.2 C at 25°C and then discharged to 3 V at a constant current of 5.0 C. The proportion of the discharge capacity at 5.0 C to the initial discharge capacity was determined, and this was defined as the discharge capacity ratio (%) at 5.0 C. (Discharge capacity at 5.0 C)/(initial discharge capacity at 0.2 C) × 100 = discharge capacity ratio (%) at 5.0 C

The results are shown in Table 1 with the value thereof in Comparative Example 1 set to 100.

### (Cycle characteristics)

At 25°C, the lithium ion secondary battery created above was CC/CV-charged to 4.2 V at a current corresponding to 1.0 C (0.1 C cut), then, discharged to 3 V at a constant current of 1.0 C. This was regarded as 1 cycle, and the initial discharge capacity was determined from the discharge capacity. The cycle was performed again, and the discharge capacity after 300 cycles was measured. The proportion of the discharge capacity after 300 cycles to the initial discharge capacity was determined, and this was defined as the capacity retention rate (%). (Discharge capacity after 300 cycles)/(initial discharge capacity at 1.0 C) × 100 = capacity retention rate (%)

The results are shown in Table 1 with the value thereof in Comparative Example 1 set to 100.

### (60°C Storage characteristics)

The battery for which the evaluation of the initial discharge capacity had been ended was CC/CV-charged again to 4.2 V (0.1 C cut), and then preserved at a high temperature under conditions of 60°C for 7 days. Next, the battery was discharged to 3 V at 0.2 C at 25°C, the remaining capacity after high-temperature preservation was measured, and the proportion of the remaining capacity to the initial discharge capacity was determined, and this was defined as the preserved capacity retention rate (%). (Remaining capacity)/(initial discharge capacity) × 100 = preserved capacity retention rate (%)

The results are shown in Table 1 with the value thereof in Comparative Example 1 set to 100.

### (Gas generation rate)

The battery for which the evaluation of the initial discharge capacity had been ended was CC/CV-charged again to 4.2 V (0.1 C cut) and then preserved at a high temperature under conditions of 60°C for 7 days. After the battery was sufficiently cooled, the volume of the battery was measured by Archimedes' principle, and the gas generation rate was determined from the volume change before and after the high-temperature preservation. Gas generation rate (%) = (volume (ml) after high-temperature preservation)/(volume (ml) before high-temperature preservation) × 100

The results are shown in Table 1 with the value thereof in Comparative Example 1 set to 100.

### Examples 2 to 4 and Comparative Examples 1 and 2

Compositions were prepared in the same manner as in Example 1 except that the kind and amount of the binder and the kind of the solvent were changed as shown in Table 1, and the viscosities of the obtained compositions were measured. The results are shown in Table 1.

Furthermore, positive electrode mixtures were prepared and positive electrodes were created in the same manner as in Example 1 except that the compositions prepared in the examples and the comparative examples were used and evaluated in the same manner as in Example 1. The results are shown in Table 1.

### [Table 1]

**Table 1**

| | | | Example 1 | Example 2 | Example 3 | Example 4 | Comparative Example 1 | Comparative Example 2 |
|---|---|---|---|---|---|---|---|---|
| Formulation of composition | | | | | | | | |
| | Solvent | | 3-Methoxy-N,N-dimethy Ipropaneamide | N-butyl-2-pyrrolidone | 3-Methoxy-N,N-dimethy Ipropaneamide | N-butyl-2-pyrrolidone | N-metyl-2-pyrrolidone | N-metyl-2-pyrrolidone |
| | Content of fluorine-containing copolymer (A) | wt% | 1.75 | 1.75 | 1.00 | 1.00 | 1.75 | 1.00 |
| | Content of PVdF (B) | wt% | - | - | 4.00 | 4.00 | - | 4.00 |
| | Content of single-walled carbon nanotube | wt% | 0.5 | 0.5 | 0.4 | 0.4 | 0.5 | 0.4 |
| | Viscosity of composition | mPa·s | 1320 | 1450 | 1820 | 1910 | 1420 | 1870 |

| Formulation of binder in positive electrode mixture | | | | | | | | |
|---|---|---|---|---|---|---|---|---|
| | IMass ratio (fluorine-containing copolymer (A)/PVdF (B)) | wt% | 22/78 | 22/78 | 20/80 | 20/80 | 22/78 | 20/80 |

| Formulation of positive electrode mixture | | | | | | | | |
|---|---|---|---|---|---|---|---|---|
| | Positive electrode active material | | NMC811 | NMC811 | NMC811 | NMC811 | NMC811 | NMC811 |
| | Content of positive electrode active material | Parts by mass | 100 | 100 | 100 | 100 | 100 | 100 |
| | Content of acetylene black | Parts by mass | 0.41 | 0.41 | 0.41 | 0.41 | 0.41 | 0.41 |
| | Content of single-walled carbon nanotube | Parts by mass | 0.08 | 0.08 | 0.08 | 0.08 | 0.08 | 0.08 |
| | Content of binder | Parts by mass | 1.00 | 1.00 | 1.00 | 1.00 | 1.00 | 1.00 |

| Battery characteristics | | | | | | | | |
|---|---|---|---|---|---|---|---|---|
| | Output characteristics: discharge capacity ratio (5.0C/0.2C) | % | 112.24 | 108.16 | 106.12 | 114.29 | 100 | 97.96 |
| | Cycle characteristics: capacity retention rate (300 cycles) | % | 107.69 | 117.95 | 108.97 | 116.67 | 100 | 97.44 |
| | 60°C storage characteristics: preserved capacity retention rate (for 7 days) | % | 114.47 | 122.37 | 114.47 | 121.05 | 100 | 102.63 |
| | Gas generation rate | % | 50.00 | 67.86 | 46.43 | 75.00 | 100 | 92.86 |

### Example 5

### (Preparation of composition)

A composition having a formulation shown in Table 2 was prepared by mixing a single-walled carbon nanotube, the fluorine-containing copolymer (A), and 3-methoxy-N,N-dimethylpropaneamide. The viscosity of the obtained composition was measured. The result is shown in Table 2.

### (Preparation of negative electrode mixture)

The obtained composition and the PVdF (B) were mixed so that the mass ratio of the fluorine-containing copolymer (A) to the PVdF (B) became as shown in Table 2. The obtained mixed solution, a graphite powder, and 3-methoxy-N,N-dimethylpropaneamide were mixed to prepare a negative electrode mixture. The formulation of the obtained negative electrode mixture is shown in Table 2.

### (Creation of negative electrode including negative electrode material layer)

The obtained negative electrode mixture was applied to a single side of a negative electrode current collector made of a copper foil and dried. This was cut into a predetermined electrode size and rolled by using a roll press, to create a negative electrode including a negative electrode material layer and the negative electrode current collector.

### (Creation of positive electrode)

97 Parts by mass of LiNi_{0.8}Mn0.1Co_{0.1}O₂ (NMC811) as a positive electrode active material, 1.5 parts by mass of acetylene black (AB) as a conductive aid, and 1.5 parts by mass of a NMP solution of 8% by mass of PVDF (B) as a binder were mixed to prepare a positive electrode mixture having a solid content concentration of 71% by mass. The obtained positive electrode mixture was uniformly applied to one side of a positive electrode current collector (aluminum foil having a thickness of 20 µm) and dried at 120°C for 60 minutes to completely volatilize N-methyl-2-pyrrolidone, and then the positive electrode current collector was pressed by applying a pressure of 10 tons by using a roll press, to create a positive electrode including a positive electrode material layer and the positive electrode current collector.

### (Creation of lithium ion secondary battery)

The positive electrode, the negative electrode, and a separator made of polyethylene were laminated in the order of the negative electrode, the separator, and the positive electrode to create a battery element.

This battery element was intercalated into a bag made of a laminate film, which was an aluminum sheet (having a thickness of 40 µm) covered with resin layers on both sides, while the terminals of the positive electrode and the negative electrode were provided to protrude. Next, 1.2 g of an electrolytic solution (an electrolytic solution obtained by dissolving LiPF₆ at a concentration of 1 mol/liter in a solvent obtained by mixing ethylene carbonate and ethyl methyl carbonate in a volume ratio of 3/7 and further adding 2% by mass of vinylene carbonate thereto) was each injected into the bag, and vacuum sealing was performed, to create a sheet-like lithium ion secondary battery.

### (Measurement of battery characteristics)

For the lithium ion secondary battery created above, the output characteristics, the cycle characteristics, the 60°C storage characteristics and the gas generation rate were evaluated in the same manner as in Example 1. The results are shown in Table 2 with the values thereof in Comparative Example 3 set to 100.

### Examples 6 to 10 and Comparative Examples 3 to 5

Compositions were prepared in the same manner as in Example 5 except that the kind and amount of the binder and the kind of the solvent were changed as shown in Table 2, and the viscosities of the obtained compositions were measured. The results are shown in Table 2.

Furthermore, negative electrode mixtures were prepared and negative electrodes were created in the same manner as in Example 5 except that the compositions prepared in the examples and the comparative examples were used and evaluated in the same manner as in Example 5. The results are shown in Table 2 with the values thereof in Comparative Example 3 set to 100.

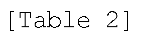

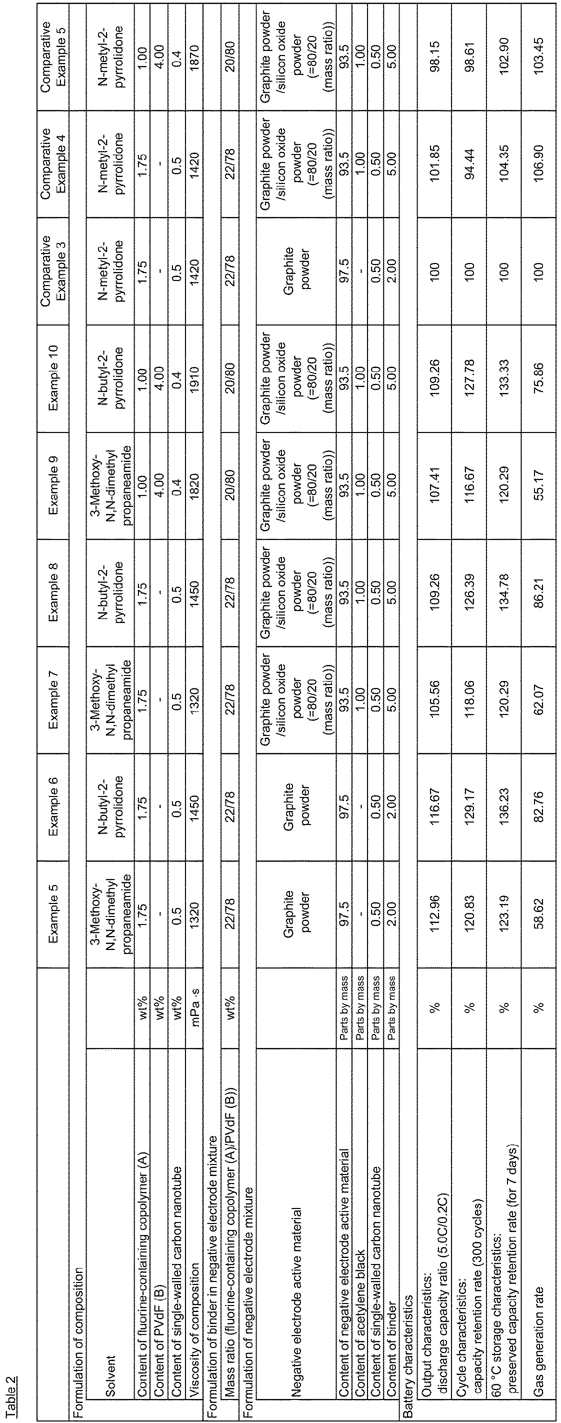

## Claims

1. A composition, comprising: a single-walled carbon nanotube; a binder; and a solvent represented by a general formula (1), wherein the binder contains a fluorine-containing polymer containing vinylidene fluoride unit, 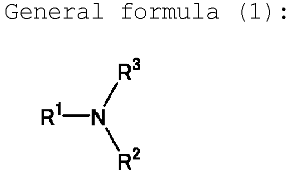 wherein R¹, R², and R³ are each independently H or a monovalent substituent, provided that the total number of carbon atoms of R¹, R², and R³ is 6 or more, and at least one of R¹, R², and R³ is an organic group having a carbonyl group; and any two of R¹, R², and R³ optionally bond to each other to form a ring.

2. The composition according to claim 1, wherein the solvent is a solvent represented by a general formula (1a): 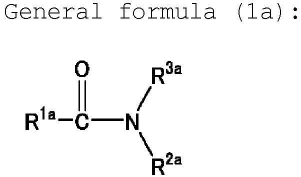 wherein R^{1a} is a monovalent substituent, R^{2a} and R^{3a} are each independently H or a monovalent substituent, provided that the total number of carbon atoms of R^{1a}, R^{2a}, and R^{3a} is 5 or more; and any two of R^{1a}, R^{2a}, and R^{3a} optionally bond to each other to form a ring.

3. The composition according to claim 1 or 2, wherein the solvent is at least one selected from the group consisting of a solvent represented by a general formula (1b-1) and a solvent represented by a general formula (1b-2): 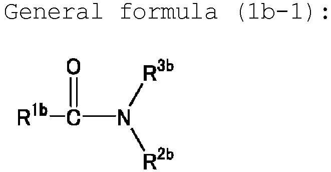 wherein R^{1b} is an alkyl group, an alkoxyalkyl group, an acylalkyl group, an alkenyl group, an amino group, or an aminoalkyl group, R^{2b} and R^{3b} are each independently an alkyl group or an alkoxyalkyl group, the total number of carbon atoms of R^{1b}, R^{2b}, and R^{3b} is 5 or more, R^{2b} and R^{3b} optionally bond to each other to form a ring with a nitrogen atom to which R^{2b} and R^{3b} bond, and an oxygen atom is optionally contained as a ring-constituting atom. 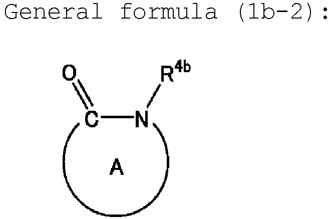 wherein a ring A is a 5-membered or 6-membered amide ring, R^{4b} is an alkyl group, a cycloalkyl group, or an alkenyl group, and the total number of carbon atoms of the ring A and R^{4b} is 5 or more.

4. The composition according to any one of claims 1 to 3, wherein the solvent is at least one selected from the group consisting of 3-methoxy-N,N-dimethylpropanamide, N-ethyl-2-pyrrolidone, and N-butyl-2-pyrrolidone.

5. The composition according to any one of claims 1 to 4, wherein an average outer diameter of the single-walled carbon nanotube is 2.5 nm or less.

6. The composition according to any one of claims 1 to 5, wherein an average G/D ratio of the single-walled carbon nanotube is 2 or more.

7. The composition according to any one of claims 1 to 6, wherein the binder contains a polyvinylidene fluoride as the fluorine-containing polymer.

8. The composition according to any one of claims 1 to 6, wherein the binder contains a fluorine-containing copolymer containing vinylidene fluoride unit and a unit of an other monomer other than vinylidene fluoride as the fluorine-containing polymer.

9. The composition according to claim 8, wherein the other monomer is a fluorinated monomer, excluding vinylidene fluoride.

10. The composition according to claim 8, wherein the other monomer is at least one selected from the group consisting of tetrafluoroethylene, chlorotrifluoroethylene, (meth)acrylic acid, 2,3,3,3-tetrafluoropropene, hexafluoropropylene, and a fluoroalkyl vinyl ether.

11. The composition according to any one of claims 8 to 10, wherein the binder contains a polyvinylidene fluoride and a fluorine-containing copolymer containing vinylidene fluoride unit and a unit of an other monomer other than vinylidene fluoride, as the fluorine-containing polymer.

12. The composition according to claim 11, wherein a mass ratio of the polyvinylidene fluoride to the fluorine-containing copolymer, polyvinylidene fluoride/fluorine-containing polymer, is 99/1 to 1/99.

13. The composition according to any one of claims 1 to 12, wherein the composition is used to form an electrode of an electrochemical device.

14. The composition according to any one of claims 1 to 13, further comprising a powder electrode material, excluding the single-walled carbon nanotube.

15. The composition according to claim 14, wherein the powder electrode material comprises a lithium composite oxide as a positive electrode active material.

16. The composition according to claim 14, wherein the powder electrode material comprises a negative electrode active material that exhibits a potential of 2.5 V or less vs. Li when alloyed with Li or bonded with Li.

17. An electrode comprising: a current collector; and an electrode material layer provided on one side or each of both sides of the current collector and formed from the composition according to any one of claims 1 to 16.

18. A secondary battery comprising the electrode according to claim 17.
